# EUROPEAN PATENT APPLICATION

(11) **EP 3 904 294 A1**
(43) Date of publication of application: **03.11.2021**
(21) Application number: 19902655.0
(22) Date of filing: 27.12.2019
(51) Int. Cl.: C01G 49/00, C01G 49/02, C01G 49/06, C01G 49/08, A61K 49/06, B01J 31/02

(54) **METHOD FOR PRODUCING NANOPARTICLES INCLUDING METAL PARTICLES CONTAINING IRON OXIDE HAVING AT LEAST ONE HYDROPHILIC LIGAND COORDINATED THERETO**

(30) Priority: 27.12.2018 JP 2018243905; 27.12.2018 JP 2018243906
(71) Applicant: Astellas Pharma Inc., Chuo-ku Tokyo 103-8411 (JP)
(72) Inventor: KIKUCHI, Takashi, Tokyo 103-8411 (JP); MIZUTANI, Tsuyoshi, Tokyo 103-8411 (JP); TOYA, Hiroki, Tokyo 103-8411 (JP); KURODA, Akio, Tokyo 103-8411 (JP); GOTO, Yota, Tokyo 103-8411 (JP); HIRASAWA, Shun, Tokyo 103-8411 (JP); SUGIMORI, Toshiyuki, Tokyo 103-8411 (JP)
(74) Representative: Hoffmann Eitle
(86) International application number: PCT/JP2019/051386
(87) International publication number: WO 2020/138404

(57) **Abstract**

[Problem]

A novel method for producing a nanoparticle having a metal particle which contains iron oxide to which one or more hydrophilic ligands are coordination bonded is provided, where the nanoparticle is useful as a contrast agent for magnetic resonance imaging.

[Means for Solution]

As the novel method for producing a nanoparticle having a metal particle which contains iron oxide to which one or more hydrophilic ligands are coordination bonded, by performing ligand exchange to a hydrophilic ligand from an iron oxide nanoparticle having a surface to which a hydrophobic ligand is coordination bonded in one step using a phase transfer catalyst, it is possible to expect shortening of production processs and reduction of hydrophilic ligands used.

Furthermore, by producing an iron oxide nanoparticle having a surface to which a hydrophobic ligand is coordination bonded using a dropwise addition method, it is possible to avoid a rapid temperature rise and a reaction at a high temperature of 200°C or higher, which is more advantageous for industrial production.

## Description

### Technical Field

The present invention relates to a method for producing a nanoparticle having a metal particle which contains iron oxide to which one or more hydrophilic ligands are coordination bonded, where the nanoparticle is useful as a contrast agent for magnetic resonance imaging, and the like.

### Background Art

Magnetic resonance imaging (MRI) plays an important role in clinical image diagnosis, and is also an important tool in the field of biomedical research.

Gadolinium (Gd)-based chelate and gadolinium oxide nanoparticles have been used clinically as positive contrast agents of MRI, but Gd-based compounds are known to exhibit toxicity to the elderly and patients with renal insufficiency. Therefore, in recent years, research and development of iron oxide-based nanoparticles having extremely low toxicity have been promoted (Non-Patent Document 1).

Recently, a method has been developed for synthesizing iron oxide nanoparticles having a surface to which an oleophilic (hydrophobic) surfactant is coordination bonded by rapidly heating iron precursors (for example, iron complexes of C₁₀₋₂₂ fatty acids) or iron pentacarbonyl to a high temperature in an organic solvent together with a surfactant such as C₁₀₋₂₂ fatty acid and the like, and/or by thermally decomposing the iron precursors or the iron pentacarbonyl at a high temperature of 200°C to 350°C for a certain time or longer (Patent Document 1; Patent Document 2; and Non-Patent Document 2). This method is advantageous in adjusting the size and shape of nanoparticles, and the synthesized nanoparticles have the advantages of being extremely uniform in size, excellent in crystallinity, and free of agglomeration. However, the synthesized nanoparticles have a drawback that they are not dispersed in water because a surfactant having hydrophobicity is adhered to the surface, and it is difficult to use them in a biological body as a contrast agent and the like. Accordingly, in order to use iron nanoparticles produced in this manner in a biological body, it is required to surface-modify them to be hydrophilic. As a method for surface-modifying hydrophobic nanoparticles to be hydrophilic, a ligand exchange method, in which hydrophobic ligands bonded to the surface of a nanoparticle are removed and hydrophilic ligands are introduced instead of the hydrophobic ligands, and the like are performed.

For example, a method has been reported in which iron oxide nanoparticles to which oleic acid is coordination bonded, and an excessive amount of hydrophilic ligands are heated and reacted in a mixed solution of an aqueous solvent and an organic solvent to perform ligand exchange (Patent Document 3; and Patent Document 1). However, there were disadvantages that the method is complicated and the yield is low, because it is required to devise selection of a hydrophilic ligand that is easily dispersed in an organic solvent, dispersion of nanoparticles in water to use them in the reaction, and the like (Patent Document 4).

Furthermore, a method has been reported in which iron oxide nanoparticles from which hydrophobic ligands are removed are obtained by annealing a powder mixture containing iron oxide nanoparticles having hydrophobic ligands on the surface, and salt particles, and then hydrophilic ligands are coated onto the surface (Patent Document 4). However, it is required to perform annealing at a high temperature of around 500°C.

Recently, iron nanoparticles, which have zwitterionic ligands, useful for T1-enhanced MRI imaging have been reported (Non-Patent Document 3; Patent Document 2; and Patent Document 5). As a method for producing the same, a two-step method is disclosed in which first, iron oxide nanoparticles to which oleic acid is coordination bonded are ligand-exchanged with 2-[2-(2-methoxyethoxy)ethoxy]acetic acid (MEAA), iron oxide nanoparticles to which MEAA is coordination bonded are separated, and then, in a mixture of N,N-dimethylformamide (DMF) and water, MEAA is ligand-exchanged with a zwitterionic ligand, and thereby iron oxide nanoparticles to which zwitterionic ligands are bonded are obtained. This method facilitated introduction of hydrophilic ligands (for example, zwitterionic ligands) which have low affinity for organic solvents.

Furthermore, as another two-step method, a method is disclosed in which first, ultra-small superparamagnetic iron oxide nanoparticles (USPIONs) to which oleic acid is coordination bonded are reacted with tetramethylammonium hydroxide (TMAOH), which is a strong base, in a mixed solvent of chloroform-water so that oleic acid is ligand-exchanged with TMAOH to ensure water solubility, and then this TMAOH ligand is ligand-exchanged with peptide ligands having a phosphoric acid group and L-3,4-dihydroxyphenylalanine (DOPA), and thereby USPIONs having a surface to which various peptide ligands are coordination bonded are obtained (Non-Patent Document 4).

However, the method for producing iron oxide nanoparticles disclosed in Patent Document 2 or 5 or Non-Patent Document 3 requires two steps, and furthermore, purification by centrifugal separation is required in all steps. Therefore, further improvement was desired in the method in terms of industrial production.

Furthermore, the method for producing nanoparticles disclosed in Non-Patent Document 4 requires two steps, and furthermore, the aqueous solution of TMAOH is strongly basic. Therefore, the method is not suitable for industrial production also from the viewpoint of safety.

### Related Art

### Patent Document

[Patent Document 1] International Publication No. WO2012/018240
[Patent Document 2] International Publication No. WO2013/090601
[Patent Document 3] International Publication No. WO2013/019090
[Patent Document 4] International Publication No. WO2012/108648
[Patent Document 5] International Publication No. WO2016/044068

### Non-Patent Document

[Non-Patent Document 1] Claire Corot et al., Advanced Drug Delivery Reviews, 58, 1471-1504, 2006
[Non-Patent Document 2] Jongnam Park et al., Nature Mater., 3, 891-895, 2004
[Non-Patent Document 3] He Wei et al., Nano Lett., 12, 22-25, 2012
[Non-Patent Document 4] Heng Li Chee et al., ACS Nano, 12, 6480-6491, 2018

### Disclosure of Invention

### Problems to Be Solved by the Invention

It is desired to develop a method for producing a nanoparticle having a metal particle which contains iron oxide to which one or more hydrophilic ligands are coordination bonded, where the nanoparticle is expected to be used as an MRI contrast agent and the like.

### Means for Solving the Problems

The present invention relates to a method for producing a nanoparticle having a metal particle which contains iron oxide to which one or more hydrophilic ligands are coordination bonded, where the nanoparticle is expected to be highly useful as a novel MRI contrast agent.

The present invention relates to a method for producing a nanoparticle having a metal particle which contains iron oxide to which one or more hydrophilic ligands are coordination bonded (hereinafter, may be abbreviated as a phase transfer catalyst method), the method including reacting an iron oxide nanoparticle having a surface to which a hydrophobic ligand is coordination bonded, with a hydrophilic ligand in a two-layer solvent of an organic layer and an aqueous layer in the presence of a phase transfer catalyst to exchange a ligand to be bonded from a hydrophobic ligand to a hydrophilic ligand.

The present invention further relates to a method for producing a nanoparticle having a metal particle which contains iron oxide to which one or more hydrophilic ligands are coordination bonded, in which the hydrophobic ligand is a C₁₀₋₂₂ fatty acid, and the hydrophilic ligand is a zwitterionic ligand shown by the formula (I).

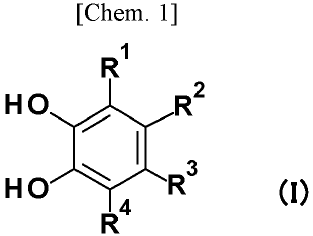

(In the formula,
one of R¹ and R² is a group shown by the formula (a) or the formula (b), and the other is H, lower alkyl, O-lower alkyl, or halogen, 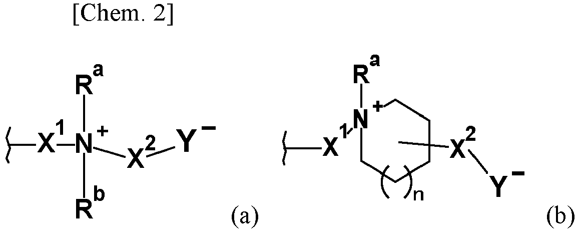
X¹ is a bond or methylene, and when R¹ is the group shown by the formula (a), X¹ may be ethylene,
X² is C₁₋₅ alkylene which may be substituted with OH, or C₁₋₂ alkylene-O-C₁₋₃ alkylene, and when R¹ is the group shown by the formula (b), X² may be a bond,
R^{a} and R^{b} are the same as or different from each other and each are C₁₋₃ alkyl or C₁₋₃ alkylene-O-C₁₋₂ alkyl, or R^{a} and R^{b} are integrated with a quaternary nitrogen atom to which R^{a} and R^{b} are bonded to form a 5- or 6-membered nitrogen-containing saturated heterocycle,
Y⁻ is SO₃⁻, HPO₃⁻, or CO₂⁻,
R³ and R⁴ are the same as or different from each other and each are H, C₁₋₃ alkyl, O-C₁₋₃ alkyl, or halogen,
n is an integer of 0 to 2, and

i) when R¹ is the group shown by the formula (a) and X¹ is methylene, R² and R^{a} or R^{b} may be integrated with each other to form ethylene,
ii) when R¹ is the group shown by the formula (a) and X¹ is ethylene, R² and R^{a} or R^{b} may be integrated with each other to form methylene, and
iii) when R² is the group shown by the formula (a) and X¹ is methylene, R³ and R^{a} or R^{b} may be integrated with each other to form ethylene.)

The present invention relates to a method for producing a nanoparticle consisting of iron oxide (iron oxide nanoparticle) having a surface to which a hydrophilic ligand, which is a zwitterionic ligand shown by the formula (Ia), is coordination bonded, the method including reacting an iron oxide nanoparticle having a surface to which a hydrophobic ligand, which is a C₁₀₋₂₂ fatty acid, is coordination bonded, with a hydrophilic ligand, which is the zwitterionic ligand shown by the formula (Ia), in a two-layer solvent of an organic layer and an aqueous layer in the presence of a phase transfer catalyst to exchange a ligand to be bonded from a hydrophobic ligand to a hydrophilic ligand.

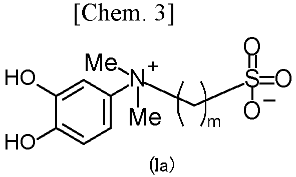

(In the formula, m represents an integer of 1 to 4.)

The present invention relates to a method for producing a nanoparticle having a metal particle which contains iron and/or iron oxide and which has a surface to which one or more zwitterionic ligands are coordination bonded, the method including reacting an iron oxide nanoparticle to which a fatty acid ligand is coordination bonded, with a zwitterionic ligand shown by the formula (I) in a two-layer solvent of an organic layer and an aqueous layer in the presence of a phase transfer catalyst.

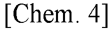

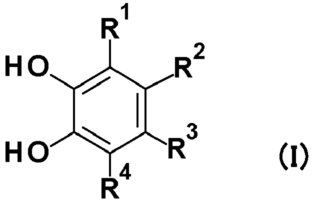

(In the formula,
one of R¹ and R² is a group shown by the formula (a), and the other is H, lower alkyl, O-lower alkyl, or halogen, 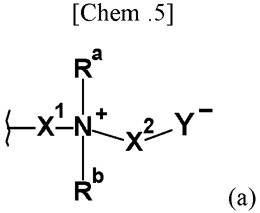
X¹ is a bond or methylene, and when R¹ is the group shown by the formula (a), X¹ may be ethylene,
X² is C₁₋₅ alkylene which may be substituted with OH, or C₁₋₂ alkylene-O-C₁₋₃ alkylene,
R^{a} and R^{b} are the same as or different from each other and each are C₁₋₃ alkyl or C₁₋₃ alkylene-O-C₁₋₂ alkyl, or a quaternary nitrogen atom and R^{a} and R^{b} bonded to the quaternary nitrogen atom may be integrated with each other to form nitrogen-containing heterocycloalkyl,
Y⁻ is SO₃⁻, HPO₃⁻, or CO₂⁻,
R³ and R⁴ are the same as or different from each other and each are H, C₁₋₃ alkyl, O-C₁₋₃ alkyl, or halogen, and
when R¹ is the group shown by the formula (a),
   i) X¹ is methylene, and R² and R^{a} or R^{b} may be integrated with each other to form ethylene, and
   ii) X¹ is ethylene, and R² and R^{a} or R^{b} may be integrated with each other to form methylene, or
when R² is the group shown by the formula (a), X¹ is methylene, and R³ and R^{a} or R^{b} may be integrated with each other to form ethylene,
where when R² is the group shown by the formula (a), R^{a} and R^{b} are methyl, X¹ is a bond, X² is C₁₋₄ alkylene, and R¹, R³, and R⁴ are all H, Y⁻ is HPO₃⁻ or CO₂⁻.)

Furthermore, the present invention relates to a method for producing an iron oxide nanoparticle having a surface to which a C₁₀₋₂₂ fatty acid is coordination bonded (hereinafter, may be abbreviated as a dropwise addition method), the method including:
i) preparing a first solution at 150°C to 190°C, and a second solution at 0°C to 120°C containing an iron precursor (iron complex of a C₁₀₋₂₂ fatty acid), where any one or both of the first solution and the second solution contain one or more surfactants selected from the group consisting of C₁₀₋₂₂ fatty alcohols, C₁₀₋₂₂ fatty acids, and C₁₀₋₂₂ fatty amines;
ii) maintaining the first solution at 150°C to 190°C, and dropwise adding the second solution into the first solution; and
iii) reacting the mixed solution at 150°C to 190°C.

The iron oxide nanoparticle, which has a surface to which a C₁₀₋₂₂ fatty acid is coordination bonded, produced by the production method can be used as a starting material for the phase transfer catalyst method of the present invention.

The present invention includes a method for producing a nanoparticle having a metal particle which contains iron oxide to which one or more hydrophilic ligands are coordination bonded, where the production method is a combination of the above-described dropwise addition method and the above-described phase transfer catalyst method.

### Effects of the Invention

In a production method of the present invention, it is possible to efficiently perform ligand exchange to a hydrophilic ligand from an iron oxide nanoparticle having a surface to which a hydrophobic ligand is coordination bonded in one step using a phase transfer catalyst. Thereby, it is possible to expect shortening of production processs, reduction of hydrophilic ligands used, suppression of agglomeration of nanoparticles, and an increase in yield, and advantages in industrial production are expected.

Furthermore, by adopting a method for producing an iron oxide nanoparticle having a surface to which a C₁₀₋₂₂ fatty acid ligand is coordination bonded using the dropwise addition method of the present invention to production of a starting material of the above-described phase transfer catalyst method, it is possible to avoid rapid temperature rise treatment and a reaction at a high temperature of 200°C or higher, and thereby advantages in industrial production can be expected.

### Brief Description of the Drawings

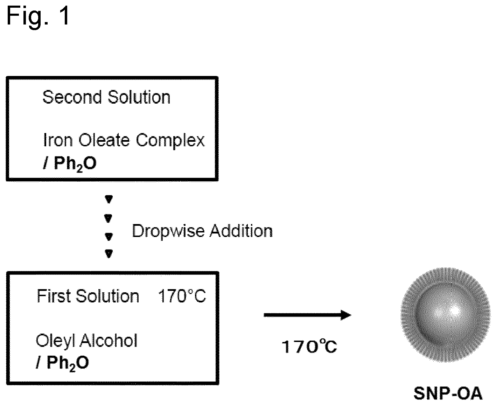

[Fig. 1] Fig. 1 is a diagram showing a production scheme for an iron oxide nanoparticle (SNP-OA) to which oleic acid was coordination bonded using a dropwise addition method described in Example 1.

### Embodiments for Carrying Out the Invention

Hereinafter, embodiments of the present invention will be described in detail.

### [Definition of terms]

"Lower alkyl" refers to linear or branched alkyl having 1 to 6 carbon atoms (hereinafter abbreviated as C₁₋₆) such as methyl, ethyl, n-propyl, isopropyl, n-butyl, isobutyl, sec-butyl, tert-butyl, n-pentyl, n-hexyl, and the like. "Lower alkyl" is linear or branched C₁₋₄ alkyl as another aspect, is linear or branched C₁₋₃ alkyl as still another aspect, is methyl, ethyl, or n-propyl as still another aspect, and is methyl as still another aspect. Furthermore, as the aspect in which "lower alkyl" is "C₁₋₃ alkyl," it is methyl, ethyl, or n-propyl, and in one aspect, "lower alkyl" is methyl.

"C₁₋₅ alkylene" means linear or branched C₁₋₅ alkylene such as methylene, ethylene, trimethylene, tetramethylene, pentamethylene, propylene, butylene, methylmethylene, ethylethylene, 1,1-dimethylethylene, 2,2-dimethylethylene, 1,2-dimethylethylene, 1-methylbutylene, and the like. "C₁₋₅ alkylene" is linear or branched C₁₋₃ alkylene as one aspect, is C₁₋₂ alkylene as another aspect, and is methylene, ethylene, trimethylene, propylene, or butylene as still another aspect. Furthermore, "C₁₋₃ alkylene" and "C₁₋₂ alkylene" are respectively linear or branched C₁₋₃ alkylene or C₁₋₂ alkylene, and in one aspect, "C₁₋₅ alkylene" is methylene or ethylene.

A "5- or 6-membered nitrogen-containing saturated heterocycle formed by integration of R^{a} and R^{b} with a quaternary nitrogen atom to which R^{a} and R^{b} are bonded" is a 5- or 6-membered non-aromatic saturated heterocycle containing a quaternary nitrogen atom as a ring-constituting atom, such as a pyrrolidine ring, a piperidine ring, or the like. As one aspect, the 5- or 6-membered nitrogen-containing saturated heterocycle is a pyrrolidine ring containing a quaternary nitrogen atom as a ring-constituting atom.

"Halogen" means F, Cl, Br, and I. "Halogen" is F, Cl, and Br in one aspect, is F in another aspect, is Cl in still another aspect, and is Br in still another aspect.

A "nanoparticle" in the specification of the present application refers to a particle having a particle diameter of nanometer scale or smaller. A "nanoparticle" refers to a particle having a particle diameter of less than 100 nm in one aspect, less than 10 nm in another aspect, less than 5 nm in still another aspect, and less than 3 nm in still another aspect. In still another aspect, a "nanoparticle" refers to a particle having a particle diameter of less than 1 nm. Details of particle diameters will be described in the following section of particle diameter.

A "cluster" in the specification of the present application refers to an assemblage in which a plurality of identical or different particles are gathered to form a single mass. In one embodiment, a "cluster" refers to an assemblage of a fine metal particle, to which a zwitterionic ligand is coordination bonded, and a zwitterionic ligand.

Hereinafter, a nanoparticle, a hydrophilic ligand, and a method for producing them in the specification of the present application will be described.

### [1. Nanoparticle]

A nanoparticle produced by a production method according to the present invention is a nanoparticle having a metal particle which contains iron oxide to which one or more hydrophilic ligands shown by the formula (I) are coordination bonded. The coordination bonded hydrophilic ligand will be described after the following sections.

In one aspect, the nanoparticle is a particle formed by coordination bonding of one or more hydrophilic ligands to an outer surface of a metal particle containing iron oxide, and is a nanoparticle in which the metal particle is coated with the hydrophilic ligands.

As one aspect, the nanoparticle is a nanoparticle which has a metal particle containing iron oxide in the central part (core) thereof, and which has a core-shell structure in which one or more hydrophilic ligands are coordination bonded to an outer surface of the metal particle and thereby the metal particle is coated with the hydrophilic ligands. In the present specification, a nanoparticle having the following core-shell structure may be written as an "iron oxide nanoparticle to which one or more hydrophilic ligands are coordination bonded," where in the core-shell structure, from an iron oxide nanoparticle having a surface to which a hydrophobic ligand is coordination bonded, only a ligand on the surface is changed to a hydrophilic ligand by ligand exchange by a phase transfer catalyst method, and iron oxide in the core does not change substantially.

In one aspect, the nanoparticle is a nanoparticle that is a complex consisting of one or more "metal particles each of which contains iron oxide to which one or more hydrophilic ligands are coordination bonded" and one or more hydrophilic ligands.

In one aspect, the nanoparticle is a nanoparticle that is a cluster consisting of two or more hydrophilic ligands, and two or more "metal particles each of which contains iron oxide to which one or more hydrophilic ligands are coordination bonded."

In one aspect, the nanoparticle is a nanoparticle that is a cluster in which two or more hydrophilic ligands, and two or more "metal particles each of which contains iron oxide to which one or more hydrophilic ligands are coordination bonded" are irregularly bonded to each other.

### (Metal particle)

A "metal particle" in the specification of the present application includes a metal particle containing iron oxide, an iron oxide particle containing only iron oxide, and a metal particle containing iron in addition to iron oxide. Furthermore, a "metal particle" also includes "iron oxide" that is a starting material and referred to in "iron oxide having a surface to which a hydrophobic ligand is coordination bonded," and includes a "metal particle containing iron oxide" in which a certain change occurred from an iron oxide nanoparticle that is a starting material as a result of performing the phase transfer catalyst method of the present invention. In this case, examples of the certain change include, but are not limited to, structural changes from core-shell structures to complexes and clusters, changes in particle diameter, changes in composition, and the like. A "metal particle" in the specification of the present application includes all metal particles containing iron oxide which are obtained when the phase transfer catalyst method of the present invention is performed using at least a zwitterionic ligand shown by the formula (I) described in the present specification.

For a metal particle used in the production method of the present invention, in one aspect, the metal particle is a metal particle containing iron oxide; in another aspect, the metal particle is an iron oxide particle containing only iron oxide; and in still another aspect, the metal particle is a metal particle containing iron in addition to iron oxide. In one aspect, the metal particle may consist only of iron oxide, and in another aspect, the metal particle may contain magnetic iron oxide. In still another aspect, the metal particle is magnetic iron oxide, and may be magnetite (Fe₃O₄), maghemite (γ-Fe₂O₃), or a mixture thereof. Such a metal particle consisting of magnetic iron oxide may be a superparamagnetic nanoparticle.

The metal particle used in the production method of the present invention may be a metal particle produced by a known method such as the methods disclosed in Patent Document 1, Non-Patent Document 2, Non-Patent Document 3 described above, and the like, or may be a commercially available metal particle. For example, the metal particle used in the production method of the present invention may be an iron oxide particle produced by a coprecipitation method or a reduction method.

In the present specification, in a case where a particle diameter is referred to, it refers to an average particle diameter unless otherwise specified.

### (Particle diameter of metal particle)

A "particle diameter" of a metal particle is intended, for example, to be a diameter of the maximum inscribed circle with respect to a two-dimensional shape of a particle in a case where the particle is observed with a transmission electron microscope (TEM). For example, in a case where a two-dimensional shape of a particle is substantially circular, a "particle diameter" is intended to be a diameter of this circle. Furthermore, in a case of a substantially elliptical shape, a "particle diameter" is intended to be a minor axis of this ellipse. Furthermore, in a case of a substantially square shape, a "particle diameter" is intended to be a length of the side of this square, and in a case of a substantially rectangular shape, a "particle diameter" is intended to be a length of the short side of this rectangle.

A method of confirming whether an average particle diameter has a value within a predetermined range can be performed by, for example, observing 100 particles with a transmission electron microscope (TEM), measuring the particle diameters of each of the particles, and obtaining an average value of the particle diameters of the 100 particles.

A particle diameter of the metal particle (including a case of an average particle diameter of a cluster or complex containing the metal particle) used in the production method of the present invention may be 5 nm or smaller, may be 4 nm or smaller, may be 3 nm or smaller, may be 2 nm or smaller, or may be 1 nm or smaller, when measured by TEM.

### (Particle diameter of nanoparticle)

A particle diameter of a nanoparticle is estimated to be larger by a thickness of a hydrophilic ligand coordination bonded to the surface of the metal particle. In general, a hydrodynamic diameter (HD) in a case where nanoparticles are in the form of a solution is used as an index of the size thereof. As an example, an average HD of nanoparticles is 10 nm or smaller, and as another example, it is 8 nm or smaller. As still another example, an average HD of nanoparticles is 5 nm or smaller; as still another example, it is 4 nm or smaller; as still another example, it is 3 nm or smaller; as still another example, it is 2 nm or smaller; and as still another example, it is 1 nm or smaller.

An HD of nanoparticles can be performed by, for example, observing the particles by a small-angle X-ray scattering (SAXS) method and obtaining an average value of the particle diameters thereof.

For measurement by SAXS, a commercially available instrument may be used, and it is desirable to use a synchrotron radiation facility such as SPring-8 (BL19B2), Aichi Synchrotron Radiation Center, and the like. For example, in a case of using SPring-8 (BL19B2), a camera length is set to 3 m, a sample is irradiated with X-rays of 18 KeV, and a wave number q is observed within a range of about 0.06 to 3 nm^{―1}.

In a case of a dispersion liquid sample, the sample is put in a capillary with a diameter of 2 mm, an exposure time is appropriately set to the extent that does not cause scattered radiation saturation, and scattered data is obtained. It is possible to obtain an average particle diameter by fitting the scattered data by Guinier analysis or using appropriate SAXS analysis software.

Furthermore, as a method of measuring relative sizes of nanoparticles, it is possible to use, for example, Size Exclusion Chromatography (SEC).

SEC is an analytical method in which a sample is poured into a column filled with a carrier having pores, and sizes of the sample are estimated by the time until it flows out. Large agglomerates flow out quickly because they do not enter the pores of the carrier, and small nanoparticles flow out slowly because they pass through the pores of the carrier and the route to flow out becomes long, and thereby relative sizes of the nanoparticles can be measured using standard particles.

An iron oxide nanoparticle having a surface to which a hydrophobic ligand is coordination bonded may be an iron oxide nanoparticle produced by a known method disclosed in Patent Document 2 described above; Byung Hyo Kim et al., J Am. Chem. Soc., 133, 12624-12631, 2011; He Wei et al., Integr. Biol., 5, 108-114, 2013; and the like, or may be a commercially available iron oxide nanoparticle. As one aspect, the iron oxide nanoparticle is an iron oxide nanoparticle, which has a surface to which a C₁₀₋₂₂ fatty acid ligand is coordination bonded, produced by the dropwise addition method described in the specification of the present application.

### (Hydrophobic ligand)

In the present invention, a "hydrophobic ligand" is a ligand having low affinity for water. As one aspect of a hydrophobic ligand used in the production method of the present invention, the hydrophobic ligand is a C₁₀₋₂₂ fatty acid that is a surfactant used in production of metal particles. As another aspect, the hydrophobic ligand is oleic acid and/or stearic acid. As still another aspect, the hydrophobic ligand is oleic acid.

### (Hydrophilic ligand)

In the present invention, a "hydrophilic ligand" is a ligand that has high affinity for water and is applicable to a biological body, and includes known hydrophilic ligands of iron oxide particles used for a contrast agent and the like. Examples thereof include dopamine sulfonate, zwitterionic dopamine sulfonate ligands (Patent Document 2); phospholipid polyethylene glycol, polyethylene glycol-phosphate, monosaccharide phosphate, or derivatives thereof, citric acid, betaine (Patent Document 1) and the like; and the like.

One aspect of a hydrophilic ligand used in the production method of the present invention is a zwitterionic ligand.

Examples of known zwitterionic ligands include ligands having a silane-functionalized zwitterionic moiety disclosed in WO2010/076237 and US7462446, betaine disclosed in Patent Document 1, and dopamine sulfonate and ligands having zwitterionic dopamine sulfonate disclosed in Non-Patent Document 3 and Patent Document 2. These known zwitterionic ligands can be used as the hydrophilic ligand in the production method of the present invention.

In the specification of the present application, a "zwitterionic ligand" is a ligand having a "zwitterionic moiety." The term "zwitterionic moiety" herein refers to a partial structure that is electrically neutral but has positive and negative formal charges on different atoms. Zwitterions are polar and are generally highly soluble in water and sparingly soluble in most organic solvents. In some aspects, the "zwitterionic moiety" also includes a precursor of the zwitterionic moiety. In such embodiments, the precursor undergoes a secondary or subsequent chemical reaction and generates a zwitterionic moiety. In one aspect, a zwitterionic moiety includes a positively charged moiety, a negatively charged moiety, and a spacer located between a positively charged moiety and a negatively charged moiety. A positively charged moiety can be formed from an organic base, and a negatively charged moiety can be formed from organic acid.

Examples of zwitterionic ligands include zwitterionic ligands in which a positively charged moiety consists of an ammonium cation, an amidinium cation, a guanidinium cation, a pyridinium cation, which are shown by the following formula, or a combination thereof, a negatively charged moiety consists of a carboxylate anion, a sulfonate anion, a sulfinate anion, a phosphonate anion, a phosphate anion, a phosphinate anion, which are shown by the following formula, or a combination thereof, and a spacer consists of alkylene (C₁₋₁₀ alkylene and the like), arylene (phenylene and the like), ether (C₁₋₅ alkylene-O-C₁₋₅ alkylene and the like), or a combination thereof; and the like.

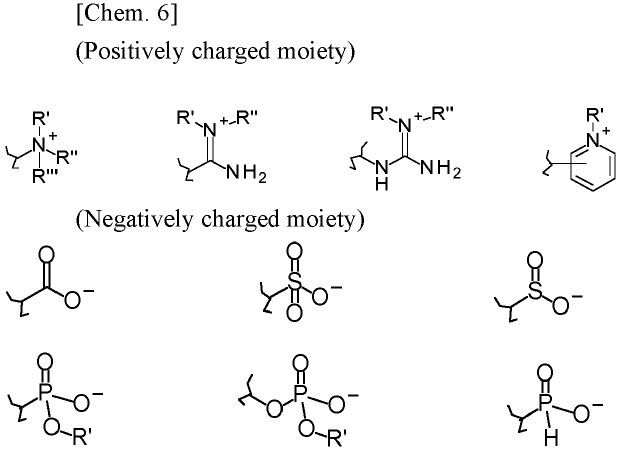

(In the formula, R' represents H or alkyl; R'' and R''' are the same as or different from each other and each represent H, alkyl, aryl, catechol, or the above-mentioned spacer; or R'' and R''' each represent a nitrogen-containing saturated heterocycle formed by being integrated with a quaternary nitrogen atom to which they are bonded.)

As one aspect of the hydrophilic ligand used in the production method of the present invention, the hydrophilic ligand is a zwitterionic ligand containing a tertiary ammonium cation or a quaternary ammonium cation as a positively charged moiety, and containing a carboxylate anion, a sulfonate anion, a sulfinate anion, a phosphonate anion, a phosphate anion, or a phosphinate anion as a negatively charged moiety. As another aspect, the hydrophilic ligand is a zwitterionic ligand in which a spacer is C₁₋₁₀ alkylene or C₁₋₅ alkylene-O-C₁₋₅ alkylene.

As another aspect of the hydrophilic ligand used in the production method of the present invention, the hydrophilic ligand is a zwitterionic ligand having a catechol structure as a partial structure. As still another aspect, the hydrophilic ligand is a compound shown by the formula (I) or a salt thereof.

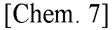

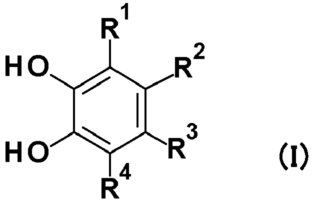

(In the formula,
one of R¹ and R² is a group shown by the formula (a) or the formula (b), and the other is H, lower alkyl, O-lower alkyl, or halogen, 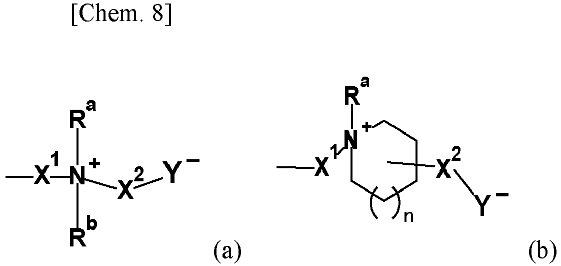
X¹ is a bond or methylene, and when R¹ is the group shown by the formula (a), X¹ may be ethylene,
X² is C₁₋₅ alkylene which may be substituted with OH, or C₁₋₂ alkylene-O-C₁₋₃ alkylene, and when R¹ is the group shown by the formula (b), X² may be a bond,
R^{a} and R^{b} are the same as or different from each other and each are C₁₋₃ alkyl or C₁₋₃ alkylene-O-C₁₋₂ alkyl, or R^{a} and R^{b} are integrated with a quaternary nitrogen atom to which R^{a} and R^{b} are bonded to form a 5- or 6-membered nitrogen-containing saturated heterocycle,
Y⁻ is SO₃⁻, HPO₃⁻, or CO₂⁻,
R³ and R⁴ are the same as or different from each other and each are H, C₁₋₃ alkyl, O-C₁₋₃ alkyl, or halogen,
n is an integer of 0 to 2, and

i) when R¹ is the group shown by the formula (a) and X¹ is methylene, R² and R^{a} or R^{b} may be integrated with each other to form ethylene,
ii) when R¹ is the group shown by the formula (a) and X¹ is ethylene, R² and R^{a} or R^{b} may be integrated with each other to form methylene, and
iii) when R² is the group shown by the formula (a) and X¹ is methylene, R³ and R^{a} or R^{b} may be integrated with each other to form ethylene.)

As still another aspect, the hydrophilic ligand is a compound shown by the formula (Ia), which is a zwitterionic ligand having a catechol structure as a partial structure, or a salt thereof. As still another aspect, the hydrophilic ligand is a compound, in which m is 3 in the formula (Ia), or a salt thereof. The formula (II) shows a state in which the compound of the formula (Ia) as a ligand is coordination bonded to a metal particle.

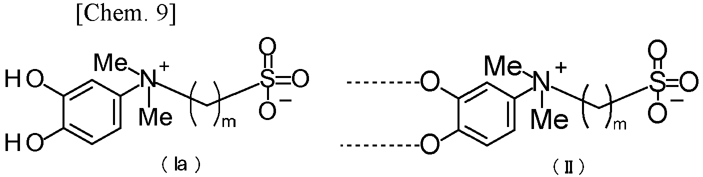

(In the formula, m is an integer of 1 to 4, and a broken line in the formula (II) represents coordination bonding with an iron atom on the surface of the metal particle, and the same shall apply hereinafter.)

(Nanoparticle having metal particle which contains iron oxide to which one or more hydrophilic ligands are coordination bonded)

One aspect of a nanoparticle produced by the production method of the present invention is a nanoparticle having a metal particle which contains iron oxide to which one or more zwitterionic ligands shown by the formula (I) above are coordination bonded as hydrophilic ligands. Furthermore, each of nanoparticles having metal particles which contain iron oxide to which the zwitterionic ligands of each of the above-described aspects are coordination bonded is one aspect of the nanoparticle produced by the production method of the present invention. In a case where a zwitterionic ligand having a catechol structure as a partial structure is coordination bonded to a metal particle containing iron oxide, as shown by the formula (II) above, oxygen of two hydroxyl groups of the catechol structure and a metal atom on the surface of the metal particle are coordination bonded to form the nanoparticle of the present invention.

The number of molecules of hydrophilic ligands coordinated on the surface of a metal particle varies depending on the size, surface area of the metal particle, and the like. In a case where the hydrophilic ligand is a zwitterionic ligand, as one aspect of the present invention, the number of ligands bonded to a metal particle is 2 to 200 per metal particle; as another aspect, the number thereof is 5 to is 50; and as still another aspect, the number thereof is 5 to 20.

### (Method for producing hydrophilic ligand)

For a hydrophilic ligand, a commercially available hydrophilic ligand may be used, and it can also be produced using the method known from the above-described documents and the like or a method known to those skilled in the art in an appropriate combination. For example, a hydrophilic ligand can be produced with reference to the method disclosed in Non-Patent Document 3.

For example, the compound of the formula (Ia) can be produced by a reaction of the following scheme.

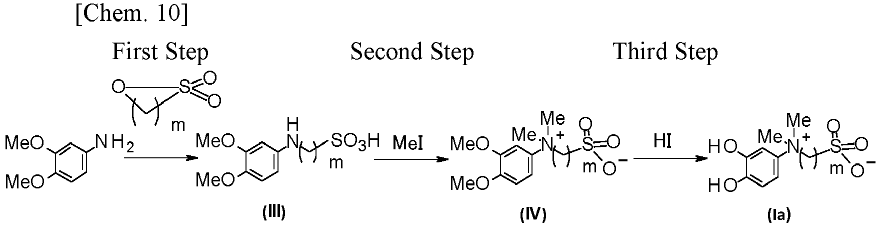

A first step is a step of producing a compound (III) by causing sultone to act on 3,4-dimethoxyaniline and subjecting it to an alkylation reaction. The reaction can be carried out by adding sultone to a mixture of 3,4-dimethoxyaniline and a solvent inert to the reaction, and stirring under cooling to heating generally for 1 hour to 3 days. Examples of solvents used herein include, but are not limited to, tetrahydrofuran (THF), dioxane, DMF, acetonitrile, and mixtures thereof.

A second step is a step of producing a compound (IV) by causing a base and a methylating agent to act on the compound (III) and subjecting it to a methylation reaction. The compound (III) is reacted with 2 equivalents to 10 equivalents of a methylating agent in a solvent inert to the reaction in the presence of 2 equivalents to 10 equivalents of a base under heating from room temperature generally for 1 hour to 24 hours. Examples of solvents used herein include, but are not limited to, methanol, THF, dioxane, DMF, acetonitrile, and mixtures thereof. Examples of bases are not particularly limited, and examples thereof include potassium carbonate, sodium carbonate, 2,6-lutidine, N,N-diisopropylethylamine, N-methylmorpholine, diazabicycloundecene (DBU (registered trademark)), diethylaniline, and dimethylaminopyridine (DMAP).

A third step is a step of producing a compound (Ia) by causing an acid to act on the compound (IV) and subjecting it to a demethylation reaction. The reaction is generally carried out for 1 hour to 24 hours under heating from room temperature. Examples of acids used herein include, but are not limited to, hydrobromic acid and hydriodic acid.

### [2. Method for producing nanoparticle by phase transfer catalyst method]

A "phase transfer catalyst" in the present specification refers to a salt which has a quaternary ammonium or phosphonium and is soluble in both an organic solvent and water. Examples of counter anions that form a salt include, but are not limited to, halide ions, hydroxide ions, hydrogen sulfate ions, and the like. For example, the phase transfer catalyst may be a phase transfer catalyst disclosed in Arthur W. Herriott et al., J. Am. Chem. Soc., 97, 2345-2349, 1975, or may be a commercially available phase transfer catalyst. As one aspect of a phase transfer catalyst used in the present invention, the phase transfer catalyst is a phase transfer catalyst selected from the group consisting of quaternary ammonium salts and quaternary phosphonium salts. As another aspect, the phase transfer catalyst is a quaternary ammonium salt selected from the group consisting of a tetramethylammonium salt, a tetrabutylammonium salt, a benzyltriethylammonium salt, a benzyltributylammonium salt, a trioctylmethylammonium salt, and a benzyldimethyloctadecylammonium salt; or a quaternary phosphonium salt selected from the group consisting of a tetrabutylphosphonium salt and a tetraphenylphosphonium salt. As still another aspect, the phase transfer catalyst is the above-mentioned quaternary ammonium salt. As still another aspect, the phase transfer catalyst is a quaternary ammonium salt selected from the group consisting of a tetrabutylammonium salt, a trioctylmethylammonium salt, a benzyldimethyloctadecylammonium salt, and a benzyltributylammonium salt. As still another aspect, the phase transfer catalyst is a quaternary ammonium salt selected from the group consisting of tetrabutylammonium halides, and benzyltributylammonium halides. As still another aspect, the phase transfer catalyst is a quaternary ammonium salt selected from the group consisting of tetrabutylammonium bromide (TBAB), tetrabutylammonium chloride, tetrabutylammonium fluoride (TBAF), and benzyltributylammonium bromide. The above-described quaternary ammonium salts and quaternary phosphonium salts may form a hydrate.

The "two-layer solvent of an organic layer and an aqueous layer" is a mixed solvent that is separated into two layers of an organic solvent and water at room temperature. The number of organic solvents may be two or more, and it is sufficient for the solvent to be separated into two layers by adding water. In one aspect, the organic solvent is selected from the group consisting of 2-methyltetrahydrofuran (2-Me-THF), cyclopentyl methyl ether (CPME), methyl tert-butyl ether (MTBE), diphenyl ether, chloroform, toluene, xylene, heptane, ethyl acetate, methyl acetate, isopropyl acetate, oleic acid, oleyl alcohol, methanol, ethanol, 1-propanol, 2-propanol (IPA), 1-butanol, 2-butanol, isobutanol, tert-butyl alcohol, 2-methyl-2-butanol, and combinations of them. As another aspect, the organic solvent is selected from 2-Me-THF, chloroform, 1-butanol, heptane, and combinations of them.

Depending on the type of hydrophilic ligand, it may be preferable to carry out a reaction further in the presence of a pH adjuster. The "pH adjuster" that can be used in the present reaction is sodium hydrogen carbonate, sodium carbonate, potassium hydrogen carbonate, ammonium hydrogen carbonate, or dipotassium hydrogen phosphate. In one aspect, the pH adjuster is sodium hydrogen carbonate.

The reaction in the phase transfer catalyst method of the present invention is carried out by stirring an iron oxide nanoparticle having a surface to which a hydrophobic ligand is coordination bonded, and a hydrophilic ligand in the two-layer solvent of an organic layer and an aqueous layer in the presence of a phase transfer catalyst in an atmosphere of one or more inert gases selected from nitrogen and argon, at room temperature or under heating, at 20°C to 80°C in one aspect, or at 30°C to 60°C in another aspect, for 1 hour or longer, for 1 to 15 hours in another aspect, and for 1 to 7 hours in still another aspect. A reaction temperature and a reaction time can be appropriately adjusted according to the type of iron oxide nanoparticle used, which has a surface to which a hydrophobic ligand is coordination bonded, and hydrophilic ligand.

In the present reaction, a hydrophilic ligand can be used at a ratio of 1 to 10 wt (weight ratio), 1 to 6 wt in one aspect, and 1 to 5 wt in another aspect, with respect to an iron oxide nanoparticle having a surface to which a hydrophobic ligand is coordination bonded. The present reaction can be performed by adding the phase transfer catalyst at a ratio of 0.1 to 10 wt, 0.5 to 6 wt in one aspect, and 0.5 to 3 wt in another aspect, with respect to an iron oxide nanoparticle having a surface to which a hydrophobic ligand is coordination bonded. In a case of further using a pH adjuster, the present reaction can be performed by adding 0.1 wt to 5 wt thereof, and 0.5 wt to 2 wt thereof in one aspect, with respect to an iron oxide nanoparticle having a surface to which a hydrophobic ligand is coordination bonded.

It is possible to isolate nanoparticles, which have a metal particle containing iron oxide to which one or more of obtained hydrophilic ligands are coordination bonded, using a publicly known method such as liquid separation operation, centrifugal separation, filtration, and the like, after a temperature is lowered to room temperature after completion of the reaction.

For example, an objective substance is extracted with water one to multiple times after adding, to the reaction mixture, a single or mixed solvent of heptane, 2-Me-THF, CPME, MTBE, chloroform, toluene, xylene, ethyl acetate, methyl acetate, isopropyl acetate, methanol, ethanol, 1-propanol, IPA, 1-butanol, 2-butanol, isobutanol, tert-butyl alcohol, 2-methyl-2-butanol, water, and the like. As desired, the aqueous layer may be washed one to multiple times with an organic solvent such as heptane or the like. Subsequently, the obtained aqueous layer is subjected to tangential flow filtration (hereinafter abbreviated as TFF) one to multiple times using an ultrafiltration membrane to obtain an aqueous dispersion liquid containing nanoparticles having a desired particle diameter. As desired, the obtained dispersion liquid may be concentrated and then freeze-dried. Examples of ultrafiltration membranes used for TFF include, but are not limited to, 1 to 50K membranes (Sartocon (registered trademark), Pellicon (registered trademark)) and 100 to 1000K membranes (Sartocon (registered trademark), Pellicon (registered trademark)), and in one aspect, 5 to 30K regenerated cellulose membranes (Sartocon (registered trademark), Pellicon (registered trademark)) and 100 to 300K regenerated cellulose membranes (Sartocon (registered trademark), Pellicon (registered trademark)).

As another method, for example, an aqueous layer obtained by extracting an objective substance with water in the same manner as described above is filtered one to multiple times using an Amicon (registered trademark) cut off filter to obtain nanoparticles with a desired particle diameter. As desired, the obtained nanoparticles may be freeze-dried. TFF is performed one to multiple times using the same or different membranes selected from, for example, 1 to 100K membranes, 1 to 50K membranes in one aspect, and 1, 2, 3, 5, 10, 30, and 50K membranes in another aspect as ultrafiltration membranes used in this case, but examples thereof are not limited thereto.

In the phase transfer catalyst method of the present invention, in a case where zwitterionic ligands are used as hydrophilic ligands, there are a case in which nanoparticles are produced in which ligands are simply exchanged from hydrophobic ligands on the surface to zwitterionic ligands, and a case in which nanoparticles are produced in which metal particles in the nanoparticles are finer than metal particles used as a starting material. Both types are obtained depending on conditions. It is presumed that this is because the zwitterionic ligand of the present invention has a property of imparting changes to metal particles when the zwitterionic ligand is coordination bonded, and types of nanoparticles to be obtained differ depending on the type of zwitterionic ligand. Furthermore, types of nanoparticles to be obtained may also change depending on reaction conditions and isolation conditions.

By adjusting the type of zwitterionic ligand to be used, reaction conditions, and isolation conditions, it is possible to obtain nanoparticles having a core-shell structure and/or nanoparticles (clusters, complexes, and the like) containing fine metal particles.

One aspect of the phase transfer catalyst method of the present invention is a method for producing a nanoparticle which has a metal particle at the central part (core) thereof, and which has a core-shell structure in which one or more hydrophilic ligands are coordination bonded to an outer surface of the metal particle and thereby the metal particle is coated with the hydrophilic ligands. The nanoparticle of the present structure is obtained in a case of using, as the hydrophilic ligands, publicly known hydrophilic ligands, and zwitterionic ligands such as the compound, in which R² has the group shown by the formula (a), in the formula (I) above, the compound shown by the formula (Ia), and the like.

In a case where the phase transfer catalyst method of the present invention is carried out using the compound in which m = 3 in the formula (Ia) above as a hydrophilic ligand, it could be confirmed that nanoparticles, which have a core-shell structure in which a hydrophobic ligand coordination bonded to a metal particle is exchanged to a hydrophilic ligand, are obtained, as described in Examples 4 to 6 to be described later. Accordingly, the phase transfer catalyst method of the present invention is a method in which ligand exchange can be performed in one step by reacting iron oxide nanoparticles to which a hydrophobic ligand that is dispersed sparingly in water is coordination bonded and a hydrophilic ligand that dissolves sparingly in an organic solvent in a two-layer solvent using a phase transfer catalyst, and thereby objective nanoparticles can be efficiently produced, and production processs can be shortened and a purification step is simplified, as compared with a conventional method described in Reference Example 5. Furthermore, it is a method suitable for industrial production because an amount of hydrophilic ligands used can also be reduced as compared with the conventional method.

Another aspect of the phase transfer catalyst method of the present invention is a method for producing a nanoparticle as a minute complex consisting of one or more "metal particles which contain iron oxide to which one or more hydrophilic ligands are coordination bonded," and one or more hydrophilic ligands. Furthermore, still another aspect is a method for producing a nanoparticle as a minute cluster consisting of two or more zwitterionic ligands and two or more "metal particles which contain iron oxide to which one or more zwitterionic ligands are coordination bonded."

In a case where the zwitterionic ligand, in which R¹ has the group shown by the formula (a) or the formula (b), in the formula (I) above is used as a hydrophilic ligand, nanoparticles (for example, 3K purified particles described in Example 7-1 to be described later, and the like) in which metal particles in the nanoparticles are finer than metal particles used as starting materials are produced. This is understood to be a phenomenon derived from a zwitterionic ligand used because, in a case where a zwitterionic ligand of a certain type is used as a hydrophilic ligand, similarly, nanoparticles finer than metal particles used as starting materials (refer to Comparative Examples 1 and 2 to be described later) are produced not only in the phase transfer catalyst method of the present invention, but also in the conventional production method (two-step method using MEAA).

One aspect of the phase transfer catalyst method of the present invention is described below as an example.
<1> A method for producing a nanoparticle having a metal particle which contains iron oxide to which one or more hydrophilic ligands are coordination bonded, the method including reacting an iron oxide nanoparticle having a surface to which a hydrophobic ligand is coordination bonded, with a hydrophilic ligand in a two-layer solvent of an organic layer and an aqueous layer in the presence of a phase transfer catalyst.
<2> The production method according to <1>, in which the hydrophilic ligand is a zwitterionic ligand containing a tertiary ammonium cation or a quaternary ammonium cation as a positively charged moiety, and containing a carboxylate anion, a sulfonate anion, a sulfinate anion, a phosphonate anion, a phosphate anion, or a phosphinate anion as a negatively charged moiety.
<3> The production method according to <2>, in which the hydrophilic ligand is a zwitterionic ligand having a catechol structure as a partial structure.
<4> The production method according to <3>, in which the hydrophobic ligand is a C₁₀₋₂₂ fatty acid, and the hydrophilic ligand is a zwitterionic ligand shown by the formula (I). 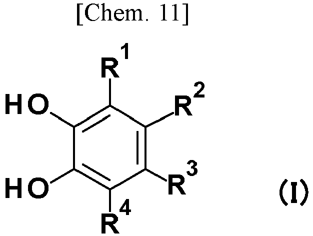 (In the formula,
   one of R¹ and R² is a group shown by the formula (a) or the formula (b), and the other is H, lower alkyl, O-lower alkyl, or halogen, 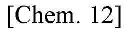 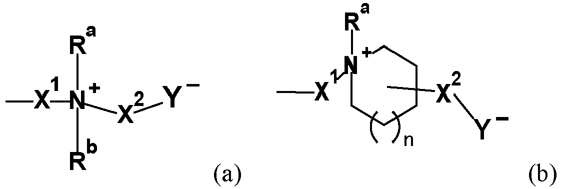
   X¹ is a bond or methylene, and when R¹ is the group shown by the formula (a), X¹ may be ethylene,
   X² is C₁₋₅ alkylene which may be substituted with OH, or C₁₋₂ alkylene-O-C₁₋₃ alkylene, and when R¹ is the group shown by the formula (b), X² may be a bond,
   R^{a} and R^{b} are the same as or different from each other and each are C₁₋₃ alkyl or C₁₋₃ alkylene-O-C₁₋₂ alkyl, or R^{a} and R^{b} are integrated with a quaternary nitrogen atom to which R^{a} and R^{b} are bonded to form a 5- or 6-membered nitrogen-containing saturated heterocycle,
   Y⁻ is SO₃⁻, HPO₃⁻, or CO₂⁻,
   R³ and R⁴ are the same as or different from each other and each are H, C₁₋₃ alkyl, O-C₁₋₃ alkyl, or halogen,
   n is an integer of 0 to 2, and
      i) when R¹ is the group shown by the formula (a) and X¹ is methylene, R² and R^{a} or R^{b} may be integrated with each other to form ethylene,
      ii) when R¹ is the group shown by the formula (a) and X¹ is ethylene, R² and R^{a} or R^{b} may be integrated with each other to form methylene, and
      iii) when R² is the group shown by the formula (a) and X¹ is methylene, R³ and R^{a} or R^{b} may be integrated with each other to form ethylene.)
<5> The production method according to <4>, in which the hydrophilic ligand is the zwitterionic ligand in which one of R¹ and R² is the group shown by the formula (a), and the other is H, lower alkyl, O-lower alkyl, or halogen.
<6> The production method according to <5>, which is a method for producing an iron oxide nanoparticle having a surface to which one or more hydrophilic ligands, which are zwitterionic ligands shown by the formula (Ia), are coordination bonded, the method including reacting an iron oxide nanoparticle having a surface to which a hydrophobic ligand, which is a C₁₀₋₂₂ fatty acid, is coordination bonded, with a hydrophilic ligand, which is a zwitterionic ligand shown by the formula (Ia), in a two-layer solvent of an organic layer and an aqueous layer in the presence of a phase transfer catalyst to exchange a ligand to be bonded from a hydrophobic ligand to a hydrophilic ligand. 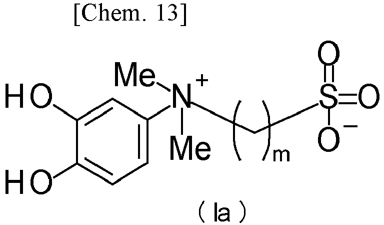 (In the formula, m is an integer of 1 to 4.)
<7> The production method according to <6>, in which the hydrophilic ligand is the zwitterionic ligand in which m is 3 in the formula (Ia).
<8> The production method according to any one of <1> to <7>, in which the phase transfer catalyst is one or more catalysts selected from the group consisting of quaternary ammonium salts and quaternary phosphonium salts.
<9> The production method according to <8>, in which the hydrophobic ligand is oleic acid and/or stearic acid, and the phase transfer catalyst is one or more catalysts selected from the group consisting of tetrabutylammonium salts, trioctylmethylammonium salts, benzyldimethyloctadecylammonium salts, and benzyltributylammonium salts.
<10> The production method according to <9>, in which the hydrophobic ligand is oleic acid, and the phase transfer catalyst is one or more catalysts selected from the group consisting of tetrabutylammonium halides and benzyltributylammonium halides.
<11> The production method according to <10>, in which the phase transfer catalyst is one or more catalysts selected from the group consisting of tetrabutylammonium bromide, tetrabutylammonium chloride, tetrabutylammonium fluoride, and benzyltributylammonium bromide.
<12> The production method according to any one of <1> to <11>, in which the iron oxide nanoparticle having a surface to which a hydrophobic ligand is coordination bonded is reacted with 1 to 10 wt (weight ratio) of hydrophilic ligands with respect to the iron oxide nanoparticle having a surface to which a hydrophobic ligand is coordination bonded, in the presence of 0.1 to 10 wt (weight ratio) of the phase transfer catalyst with respect to the iron oxide nanoparticle having a surface to which a hydrophobic ligand is coordination bonded.
<13> The production method according to any one of <1> to <12>, in which the iron oxide nanoparticle having a surface to which a hydrophobic ligand is coordination bonded is an iron oxide nanoparticle having a surface to which a C₁₀₋₂₂ fatty acid is coordination bonded and produced by a method including:
   i) preparing a first solution at 150°C to 190°C, and a second solution at 0°C to 120°C containing an iron precursor that is an iron complex of a C₁₀₋₂₂ fatty acid, where any one or both of the first solution and the second solution contain one or more surfactants selected from the group consisting of C₁₀₋₂₂ fatty alcohols, C₁₀₋₂₂ fatty acids, and C₁₀₋₂₂ fatty amines;
   ii) maintaining the first solution at 150°C to 190°C, and dropwise adding the second solution into the first solution; and
   iii) reacting the mixed solution at 150°C to 190°C.
<14> The production method according to <13>, in which the iron precursor is an iron oleate complex or an iron stearate complex.
<15> The production method according to <14>, in which the surfactant is one or more surfactants selected from the group consisting of oleyl alcohol, oleic acid, and oleylamine.
<16> The production method according to any one of <13> to <15>, in which in i), the first solution further contains a desiccant.

### [3. Method for producing iron oxide nanoparticle to which hydrophobic ligand is coordination bonded by dropwise addition method]

The present invention further relates to a production method (dropwise addition method) for an iron oxide nanoparticle having a surface to which a C₁₀₋₂₂ fatty acid is coordination bonded as a hydrophobic ligand. This dropwise addition method can be suitably used for producing nanoparticles in combination with the above-described phase transfer catalyst method.

The dropwise addition method is a method for producing an iron oxide nanoparticle having a surface to which a C₁₀₋₂₂ fatty acid is coordination bonded, the method including:
i) preparing a first solution at 150°C to 190°C, and a second solution at 0°C to 120°C containing an iron precursor that is an iron complex of a C₁₀₋₂₂ fatty acid, where any one or both of the first solution and the second solution contain one or more surfactants selected from the group consisting of C₁₀₋₂₂ fatty alcohols, C₁₀₋₂₂ fatty acids, and C₁₀₋₂₂ fatty amines;
ii) maintaining the first solution at 150°C to 190°C, and dropwise adding the second solution into the first solution; and
iii) reacting the mixed solution at 150°C to 190°C.

In the present dropwise addition method, it is advantageous for the first solution to further contain a desiccant from the viewpoint of causing the production to proceed.

The iron precursor is an iron complex of a C₁₀₋₂₂ fatty acid, is an iron oleate complex or an iron stearate complex in one aspect, and is an iron oleate complex in another aspect.

Furthermore, the C₁₀₋₂₂ fatty acid is a saturated fatty acid and an unsaturated fatty acid which have 10 to 22 carbon atoms. The C₁₀₋₂₂ fatty acid is, for example, stearic acid, oleic acid, linoleic acid, palmitic acid, palmitoleic acid, myristic acid, lauric acid, arachidonic acid, ricinoleic acid, behenic acid, or the like, and is stearic acid and/or oleic acid in one aspect. The C₁₀₋₂₂ fatty acid is oleic acid in another aspect.

The C₁₀₋₂₂ fatty alcohols are alcohols corresponding to the above-mentioned C₁₀₋₂₂ fatty acid, and are a stearyl alcohol and/or an oleyl alcohol in one aspect. Similarly, the C₁₀₋₂₂ fatty amines are amines corresponding to the above-mentioned C₁₀₋₂₂ fatty acid, and is stearylamine and/or oleylamine in one aspect.

The at least one surfactant selected from the group consisting of C₁₀₋₂₂ fatty alcohols, C₁₀₋₂₂ fatty acids, and C₁₀₋₂₂ fatty amines is one or more surfactants selected from the group consisting of the above-mentioned fatty acids, alcohols, and amines, and is one or more surfactants selected from the group consisting of stearic acid, oleic acid, stearyl alcohol, oleyl alcohol, stearylamine, and oleylamine in one aspect. As another aspect, the at least one surfactant is one or more surfactants selected from the group consisting of oleic acid, oleyl alcohol, and oleylamine. As still another aspect, the at least one surfactant is oleic acid and oleyl alcohol. As still another aspect, the at least one surfactant is oleyl alcohol. As still another aspect, the at least one surfactant is oleic acid.

The surfactant is contained in the first solution and/or the second solution. As one aspect, the surfactant is contained only in the first solution. As another aspect, the surfactant is contained only in the second solution. As still another aspect, the surfactant is contained in both of the first solution and the second solution. A surfactant in the first solution and a surfactant in the second solution may be the same as or different from each other. As one aspect, the first solution contains at least one surfactant selected from the group consisting of C₁₀₋₂₂ fatty alcohols and C₁₀₋₂₂ fatty amines, and the second solution contains a C₁₀₋₂₂ fatty acid or does not contain surfactant. As another aspect, the first solution contains oleyl alcohol and/or oleylamine, and the second solution contains oleic acid or does not contain surfactant. As still another aspect, the first solution contains oleic acid, and the second solution contains oleyl alcohol. As still another aspect, the first solution contains oleic acid and/or oleyl alcohol, and the second solution does not contain a surfactant. As still another aspect, the first solution does not contain a surfactant, and the second solution contains oleic acid and/or oleyl alcohol.

In the first solution and the second solution together, the surfactant used in the reaction is 0.1 to 10 wt, and is 0.1 to 5 wt in one aspect, with respect to the iron precursor. As an example, the first solution is 0.5 to 10 wt with respect to the iron precursor, and contains 0.5 to 5 wt of oleyl alcohol in one aspect. As another example, the second solution is 0.1 to 5 wt with respect to the iron precursor, and contains 0.1 to 2 wt of oleic acid in one aspect. In another aspect, a surfactant is not contained.

The desiccant used in the reaction is a desiccant that is inert to the reaction and is selected from the group consisting of molecular sieves, silica gel, anhydrous magnesium sulfate, and anhydrous sodium sulfate; is a molecular sieve and/or silica gel in one aspect; and is a molecular sieve (3A, 4A, 5A, and the like) in another aspect.

It is possible to use 0 to 3 wt of the desiccant with respect to the iron precursor. In one aspect, the desiccant is 0.1 to 2 wt.

Solvents used for the first solution and the second solution are solvents inert to the reaction, and both solvents may be the same as or different from each other. For example, the solvents are selected from the group consisting of octadecane, eicosane, hexadecane, tetradecene, hexadecene, octadecene, eicosene, diphenyl ether, dioctyl ether, octylphenyl ether, didecyl ether, dibenzyl ether, trioctylamine, hexadecylamine, and octadecylamine. The solvents are solvents having a boiling point of 200°C to 400°C as one aspect, is diphenyl ether or octylphenyl ether as another aspect, and is diphenyl ether as still another aspect.

The reaction can be carried out by dropwise adding a second solution, containing an iron precursor, at 0°C to 120°C, 0°C to 100°C as one aspect, or 20°C to 60°C as another aspect into a first solution at 150°C to 190°C, or 160°C to 180°C as one aspect, under the atmosphere of an inert gas selected from nitrogen and argon and in the presence of a surfactant, and if desired, a desiccant, and subsequently reacting the mixed solution at a temperature of 150°C to 190°C, or 160°C to 180°C as one aspect, for 0.1 to 48 hours, 0.25 to 12 hours as one aspect, 0.5 to 5 hours as another aspect, or 1 to 4 hours as still another aspect. The dropwise addition herein is carried out for 0.1 to 3 hours, 0.1 to 2 hours as one aspect, or 0.25 to 1 hour as another aspect, while maintaining the temperature of the first solution within the range of 150°C to 190°C, or 160°C to 180°C as one aspect.

Before the dropwise addition of the second solution, it is advantageous to stir the second solution containing an iron precursor and used in the present reaction at 70°C to 120°C, or 80°C to 100°C as one aspect, under reduced pressure, for, for example, 1 hour to 24 hours until generation of bubbles subsides (hereinafter abbreviated as degassing). In a case where the second solvent contains a surfactant, the surfactant is added before or after the degassing.

After cooling the temperature of the reaction mixture to room temperature and removing desiccants such as molecular sieves and the like, it is possible to isolate generated iron oxide nanoparticles, to which C₁₀₋₂₂ fatty acids are coordination bonded, using one of publicly known methods such as centrifugal separation, filtration, liquid separation alone, and the like or combinations thereof.

For example, a single or mixed solvent selected from the group consisting of acetone, IPA, heptane, hexane, and the like is added into the reaction mixture, and thereafter the supernatant is removed by centrifugation. This operation may be repeated. Thereafter, the obtained precipitate is separated by centrifugation by adding a single or mixed solvent such as acetone, heptane, or the like. This operation may be repeated. As desired, the obtained precipitate may be dried.

As another method, for example, a single or mixed solvent selected from the group consisting of acetone, IPA, heptane, hexane, and the like is added into the reaction mixture, and thereafter concentration is performed by circulating in a ceramic membrane filtration device. As desired, the obtained residue may be dried. Examples of ultrafiltration membranes used for ceramic membrane filtration include, but are not limited to, a membrane having a pore diameter of 2 to 5 nm.

As still another method, for example, a polar solvent and a non-polar solvent are added into the reaction mixture, and thereafter washing with the polar solvent is performed one to several times. Iron oxide nanoparticles can be isolated from the obtained organic layer on the non-polar solvent side using the above-mentioned centrifugal separation or ceramic membrane filtration method. Alternatively, in the subsequent step, the obtained organic layer on the non-polar solvent side may be used as it is, or may be used after being concentrated as a dispersion liquid containing iron oxide nanoparticles. Examples of non-polar solvents include a single or mixed solvent consisting of the group selected from heptane, hexane, 2-Me-THF, diphenyl ether, CPME, MTBE, chloroform, toluene, xylene, ethyl acetate, methyl acetate, and isopropyl acetate. Examples of polar solvents include a single or mixed solvent consisting of the group selected from water, acetone, acetonitrile, methanol, ethanol, 1-propanol, IPA, and 1-butanol. The polar solvent and the non-polar solvent used herein are preferably a combination that is separated into two layers.

In the present dropwise addition method, it is possible to produce iron oxide nanoparticles having a surface to which a hydrophobic ligand (C₁₀₋₂₂ fatty acid) is coordination bonded by gradually and rapidly increasing the liquid temperature to 150°C to 190°C by dropwise adding the solution of an iron precursor. It is shown that generated iron oxide nanoparticles tend to be small particles when a reaction time is short, and large particles when a reaction time is long. It is possible to produce iron oxide nanoparticles having a desired particle diameter by appropriately setting the type of surfactant, a reaction temperature, a reaction time, and the like.

In the present dropwise addition method, as described in Examples 1 and 3 to be described later, by gradually heating the iron precursor to around 170°C by dropwise adding it, it is possible to avoid rapid temperature rise treatment and a reaction at a high temperature of 200°C or higher, and thereby it is possible to produce iron oxide nanoparticles having a surface to which a hydrophobic ligand is coordination bonded by a method suitable for industrial production.

An embodiment of the dropwise addition method of the present invention is described below as an example.
<T-1> A method for producing an iron oxide nanoparticle having a surface to which a C₁₀₋₂₂ fatty acid is coordination bonded, the method including:
   i) preparing a first solution at 150°C to 190°C, and a second solution at 0°C to 120°C containing an iron precursor that is an iron complex of a C₁₀₋₂₂ fatty acid, where any one or both of the first solution and the second solution contain one or more surfactants selected from the group consisting of C₁₀₋₂₂ fatty alcohols, C₁₀₋₂₂ fatty acids, and C₁₀₋₂₂ fatty amines;
   ii) maintaining the first solution at 150°C to 190°C, and dropwise adding the second solution into the first solution; and
   iii) reacting the mixed solution at 150°C to 190°C.
<T-2> The production method according to <T-1>, in which the iron precursor is an iron oleate complex or an iron stearate complex, and the surfactant is one or more surfactants selected from the group consisting of oleyl alcohol, oleic acid, and oleylamine.
<T-3> The production method according to <T-1> which is characterized by including:
   i) preparing a first solution at 160°C to 180°C, and a second solution at 0°C to 100°C containing an iron oleate complex, where the first solution contains one or more surfactants selected from the group consisting of oleyl alcohol and oleylamine, and the second solution contains oleic acid or does not contain the surfactant;
   ii) maintaining the first solution at 160°C to 180°C, and dropwise adding the second solution into the first solution for 0.1 to 3 hours; and
   iii) reacting the mixed solution at 160°C to 180°C for 0.5 to 5 hours.
<T-4> The production method according to <T-3>, in which in i), the first solution contains 0.5 to 5 wt (weight ratio) of oleyl alcohol with respect to the iron oleate complex, and the second solution contains 0.1 to 2 wt (weight ratio) of oleic acid with respect to the iron oleate complex or does not contain the surfactant.
<T-5> The production method according to any one of <T-1> to <T-4>, in which in i), the first solution further contains a desiccant.

An iron oxide nanoparticle, which has a surface to which a C₁₀₋₂₂ fatty acid is coordination bonded, produced by the present dropwise addition method is suitable as a starting material used in the above-described phase transfer catalyst method, and can be used in the method for producing a nanoparticle having a metal particle which contains iron oxide to which one or more hydrophilic ligands are coordination bonded.

A commercially available iron complex can be used for the iron complex of a C₁₀₋₂₂ fatty acid which can be suitably used as an iron precursor of a starting material in the above-described dropwise addition method. Alternatively, an iron complex can be produced using known methods disclosed in the above-described documents and the like. Preferably, an iron complex can be produced with reference to the method disclosed in

### Non-Patent Document 2.

An example of producing an iron complex is described below. Iron(III) chloride hexahydrate [FeCl₃·6H₂O] and sodium oleate are dispersed in a mixture of a solvent inert to the reaction, and stirred under heating (preferably at 50°C to 80°C) for 1 to 10 hours. Subsequently, an organic layer is recovered, and washed using water, methanol-water, or ethanol-water one to multiple times. As desired, the obtained organic layer is dried and/or concentrated, and thereby an iron complex of a C₁₀₋₂₂ fatty acid can be obtained. As desired, the iron complex of a C₁₀₋₂₂ fatty acid may be stored in a state of being dispersed in the organic layer until the iron complex is used for the subsequent reaction.

In the present production method, from the viewpoint of causing the production to proceed, there are cases in which it may be advantageous to subject an iron precursor, which is the starting material of the dropwise addition method, and a metal particle, which is the starting material of the phase transfer catalyst method, to oxidation treatment in advance, and then use them in each production method.

Examples of oxidants that can be used in oxidation treatment include hydrogen peroxide solution, oxygen, air, trimethylamine N-oxide, oxone, and the like. For example, oxidation treatment of metal particles can be carried out using trimethylamine N-oxide with reference to the method disclosed in Examples of Patent Document 5.

### Examples

Hereinafter, the production methods of the present invention will be described with reference to Examples, but the present invention is not limited thereto. Furthermore, in reference examples, preparation examples, and comparative examples, a method for producing a zwitterionic ligand, and a preparation example by a publicly known production method will be described.

Furthermore, in examples, reference examples, preparation examples, and comparative examples, the following abbreviations may be used.

PEx means a preparation example number, Ex means an example number, PSyn means a preparation example number produced by the same method, Str means a chemical structural formula, and Data 1 means physicochemical data of preparation examples. NMR-D indicates δ (ppm) of a characteristic peak in ¹H-NMR in DMSO-d₆, MS indicates an m/z value in mass spectrometry (ionization method ESI, (M + H)⁺ unless otherwise noted), ESI+ indicates an m/z value in a mass spectrometric value (indicating (M + H)⁺ unless otherwise noted and indicating M⁺ in a case of ESI (M+)+ in a table shown below in the ionization method ESI), and APCI/ESI (M+)+ indicates an m/z value in a mass spectrometric value (indicating M⁺ in the ionization methods APCI and ESI). Preparation Example 27 shows Mass data of an oleic acid moiety excluding iron ions, and ESI (M-)-thereof indicates an m/z value in a mass spectrometric value (indicating M^{―} in the ionization method ESI). Data 2 means physicochemical data of examples, SEC (min) means an outflow time of nanoparticles measured in [Evaluation test of particle diameter of nanoparticles] to be described later, 3K means 3K purified particles which are particles purified by a filter described below, and 10K means 10K purified particles which are particles purified by a filter described below. THF indicates tetrahydrofuran; DMF indicates N,N-dimethylformamide; Ph₂O indicates diphenyl ether; 2-Me-THF indicates 2-methyltetrahydrofuran; OA indicates oleic acid; MEAA indicates [2-(2-methoxyethoxy)ethoxy]acetic acid; DDSA indicates (3,4-dihydroxyphenyl)(dimethyl)(3-sulfonatepropyl)ammonium; TBAF indicates tetrabutylammonium fluoride; TBAB indicates tetrabutylammonium bromide; PBS indicates phosphate buffered saline; and SNP-OA, SNP-MEAA, and SNP-DDSA respectively indicate iron oxide nanoparticles to which OA, MEAA, and DDSA are coordination bonded. Furthermore, in a structural formula, Br^{―} indicates a bromide ion, I^{―} indicates an iodide ion, Me indicates a methyl group, and Et indicates an ethyl group. For reverse phase column chromatography, a column, which was filled with silica gel having a surface modified with an octadecylsilyl (ODS) group, was used.

An Amicon (registered trademark) Ultracentrifuge 3K filter (Merck Millipore) used for purification of iron oxide nanoparticles is referred to as an Amicon (registered trademark) 3K filter. Furthermore, similarly, also in a case where the same instruments with different molecular weight cut-offs of 10K, 30K, 50K, and 100K are used, each of the instrument is referred to as an Amicon (registered trademark) 10K filter, an Amicon (registered trademark) 30K filter, an Amicon (registered trademark) 50K filter, and an Amicon (registered trademark) 100K filter. Furthermore, particles purified by ultrafiltration with molecular weight cut-offs of 30K, 10K, and 3K are respectively referred to as 30K purified particles, 10K purified particles, and 3K purified particles.

A filtration operation for particles using Agilent Captiva Premium Syringe Filters (Regenerated Cellulose, 15 mm, pore size: 0.2 µm) and YMC Duo-Filter (XQUO15, pore size: 0.2 µm) is referred to as membrane (0.2 µm) filtration.

Furthermore, broken lines in tables of the examples below represent a coordination bond with a metal atom on the surface of a metal particle.

### Reference Example 1

(3,4-dihydroxyphenyl)(dimethyl)(3-sulfonatepropyl)ammonium (hereinafter abbreviated as DDSA), which is a zwitterionic ligand, was synthesized according to Scheme 1.

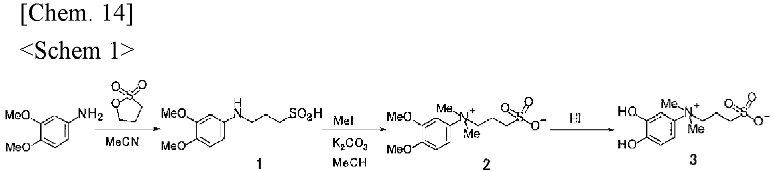

1,3-Propanesultone (13.7 mL) was added into an acetonitrile (500 mL) solution of 3,4-dimethoxyaniline (20.0 g), and the mixture was stirred at 90°C for 7 hours under an argon atmosphere. The reaction mixture was separated by filtration, washed with acetonitrile, and then dried to obtain 3-(3,4-dimethoxyanilino)propane-1-sulfonic acid (Compound 1) as a solid (31.1 g, yield 86. 5%). MS: 276

Potassium carbonate (10.7 g) and iodomethane (18.8 mL) were added into a methanol (135 mL) suspension of Compound 1 (8.90 g), and the mixture was stirred at 50°C for 18 hours under an argon atmosphere. The reaction mixture was concentrated, and purified by column chromatography (water/methanol) using a synthetic adsorbent (Sepabeads (registered trademark) SP207SS). A suspended solid, which was obtained by adding ethanol into the purified product, and heating and then cooling the mixture, was collected by filtration and dried under reduced pressure. Thereby, (3,4-dimethoxyphenyl)(dimethyl)(3-sulfonatepropyl)ammonium (Compound 2) was obtained (9.42 g, yield 96.1%). MS: 304

Compound 2 (9.71 g) was added to 57 wt% hydriodic acid (75 g), and the mixture was stirred at 120°C for 5.5 hours under an argon atmosphere. Water was added to the reaction mixture and the mixture was concentrated under reduced pressure. Thereafter, water was further added, and concentration was performed under reduced pressure twice. Water (10 mL) was added to the concentrate to dissolve it by heating. Thereafter, acetone (100 mL) was added little by little to cause precipitation. And then an organic layer was removed by decantation under ice-cooling. Water (10 mL) was added to the obtained solid, and the mixture was heated and stirred, and then cooled. Acetone (about 60 mL) was added thereto, and the mixture was stirred under ice-cooling. The precipitated solid was collected by filtration and dried under reduced pressure at 50°C to obtain DDSA (Compound 3) as a white solid (7.84 g, yield 89.0%). NMR-D: 9.85 (s, 1H), 9.52 (s, 1H), 7.18 (d, 1H, J = 3.2 Hz), 7.09 (dd, 1H, J = 8.8Hz, 3.2Hz), 6.86 (d, 1H, J = 8.8Hz), 3.82 ― 3.88 (m, 2H), 3.46 (s, 6H), 2.36 (t, 2H, J = 7.2Hz), 1.56 - 1.70 (m, 2H). MS: 276

### Reference Example 2

Iron(III) chloride hexahydrate (50 g), sodium oleate (168 g), ethanol (370 mL), water (280 mL), and heptane (650 mL) were added into a 2L three neck flask. After stirring at 70°C for 4 hours, the reaction liquid was allowed to cool to room temperature and stirred overnight. Liquid separation was performed on the reaction mixture, and the aqueous layer was removed. The organic layer was washed successively with water (365 mL), 50% methanol-water (365 mL), and water (365 mL). The organic layer was concentrated under reduced pressure at 50°C to about 335 mL. After adding 500 mL of ethanol, the operation of concentration to about 335 mL was performed twice. After adding 500 mL of heptane, concentration under reduced pressure was performed until a solution volume reached 335 mL. The obtained heptane solution was heated at 50°C for 24 hours to obtain a heptane solution (268 g) of an iron oleate complex.

### Reference Example 3

Iron(III) chloride hexahydrate (75 g), sodium oleate (251.7 g), ethanol (300 mL), water (300 mL), and heptane (525 mL) were added into a 2L three neck flask. After stirring at an internal temperature of 65°C for 4 hours, the reaction liquid was allowed to cool to room temperature and stirred overnight. The reaction mixture was transferred to a separatory funnel, and the aqueous layer was removed. The organic layer was washed successively with water (525 mL), 50% ethanol-water (526 mL), and water (525 mL).

The organic layer was concentrated under reduced pressure at 50°C to about 500 mL. After adding heptane (750 mL), concentration was performed to 503 mL or less at 50°C. The obtained heptane solution was heated at 50°C for 24 hours to obtain a heptane solution (407.1 g) of an iron oleate complex. The solution was stored as it is without purification to be used for the subsequent reaction.

Hereinafter, a preparation example of SNP-DDSA disclosed in International Publication No. WO2019/004297 will be described as a reference example.

### Reference Example 4

Oleyl alcohol (3.22 g, 12 mmol) and Ph₂O (10 g) were added to the iron oleate complex (1.8 g) under an argon atmosphere, and degassing was performed at 90°C under stirring. Thereafter, the temperature was raised to 200°C at 10°C/min., and stirring was continued at 200°C for 30 minutes. Thereafter, after lowering the temperature to room temperature, 50 mL of acetone was added, and centrifugation was performed at 8,000 rpm for 20 minutes to remove the supernatant. Chloroform was added (about 0.5 mL) until the obtained precipitate was completely dispersed, and 10 mL of acetone was further added. Thereafter, centrifugation was performed at 8,000 rpm for 20 minutes to remove the supernatant. This operation was repeated 3 times. The obtained precipitate was dried to obtain SNP-OA.

### Reference Example 5

SNP-DDSA having iron oxide nanoparticles (SNPs) having an average particle diameter of 1.8 nm as core particles was produced by a two-step method in which ligand exchange to MEAA shown in the scheme below was performed.

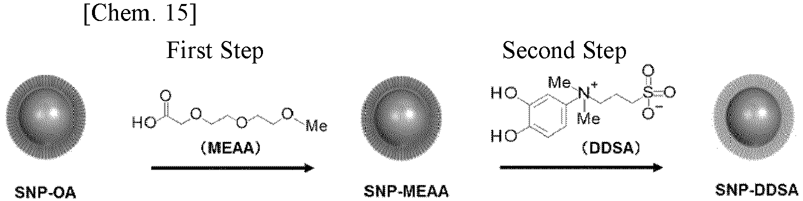

(In the formula, SNP-OA, SNP-MEAA, and SNP-DDSA schematically respectively represent iron oxide nanoparticles to which OA, MEAA, and DDSA are coordination bonded, each having SNP as a core particle, and the same shall apply hereinafter.)

### First step

Under an argon atmosphere, 10 mg of SNP-OA was dispersed in 0.9 mL of methanol, 0.1 mL of MEAA was added, and the mixture was stirred at 70°C for 4 hours. After lowering the temperature of this solution to room temperature, 8 mL of acetone and 2 mL of hexane were added, and the mixture was centrifuged at 5,800 rpm for 3 minutes to remove the supernatant liquid. This operation was repeated 3 times. The obtained precipitate was dried to obtain SNP-MEAA. Furthermore, 300 µL of water and 600 µL of DMF were added to the SNP-MEAA to prepare an SNP-MEAA solution.

### Second step

85 mg of DDSA was added into 1 mL of the SNP-MEAA solution under an argon atmosphere, and the mixture was stirred at 50°C for 12 hours. Thereafter, after lowering the temperature to room temperature, 20 mL of acetone was added, and the mixture was centrifuged at 5,800 rpm for 3 minutes to remove the supernatant. The obtained precipitate was dispersed in 2 mL of phosphate buffered saline (PBS). The obtained solution was centrifuged at 8,000 rpm for about 30 minutes using an Amicon (registered trademark) 3K filter to reduce the volume to about 1/5. PBS was added thereto so that a total volume of the solution was about 2 mL, and centrifugation was performed. This operation was repeated about 5 to 8 times until the color of the solution falling from the filter disappeared completely. The obtained solution was diluted with PBS so that a volume was 1 to 1.5 mL, and thereby an SNP-DDSA solution was obtained.

The obtained SNP-DDSA solution was stored at 4°C. As a result of observation by TEM, it was estimated that an average particle diameter of the SNP, which was the core, in the obtained SNP-DDSA was 1.8 nm from an average value of particle diameters of 100 SNPs.

Compounds of preparation examples shown in the tables below were produced by the following preparation examples or by the same method as in these preparation examples. Structural formulas and physicochemical data of these compounds are also shown in the table below.

### Preparation Example 1

A 9.5 mol/L dimethylamine aqueous solution (7.1 mL) was added to 6-fluoro-2,3-dimethoxybenzaldehyde (2.50 g), and the mixture was stirred at room temperature for 15 hours. Sodium borohydride (514 mg) was added thereto in a water bath, and the mixture was stirred at room temperature for 2 hours. Concentrated hydrochloric acid (pH of 1 to 2) was added in an ice bath. The aqueous layer was cleaned twice with dichloromethane. A 1 mol/L sodium hydroxide aqueous solution was added to this aqueous layer (pH > 11). This mixture was extracted three times with dichloromethane, and dried over anhydrous sodium sulfate. Concentration was performed after filtration, and thereby 1-(6-fluoro-2,3-dimethoxyphenyl)-N,N-dimethylmethanamine (2.46 g) was obtained.

### Preparation Example 2

Sodium triacetoxyborohydride (3.74 g) was added to a mixture of 4-fluoro-2,3-dimethoxybenzaldehyde (2.50 g), dichloromethane (75 mL), a 2 mol/L dimethylamine THF solution (13.6 mL) in a water bath, and the mixture was stirred at room temperature for 1 hour. Basic silica gel was added, and concentration was performed under reduced pressure. Purification was performed by basic silica gel column chromatography (developing solvent: hexane-chloroform), and thereby 1-(4-fluoro-2,3-dimethoxyphenyl)-N,N-dimethylmethanamine (2.81 g) was obtained.

### Preparation Example 3

A mixture of 1-(2,3-dimethoxyphenyl)-N,N-dimethylmethanamine (3.82 g), 1,2λ⁶-oxathiolane-2,2-dione (1.89 mL), and ethyl acetate (38.2 mL) was stirred at room temperature for 7 days. 1,2λ⁶-oxathiolane-2,2-dione (515 µL) was further added, and the mixture was stirred at 50°C for 4 hours. After allowing the mixture to cool to room temperature, the solid was collected by filtration, cleaned with ethyl acetate, and dried under reduced pressure. Thereby, 3-{[(2,3-dimethoxyphenyl)methyl](dimethyl)azaniumyl}propane-1-sulfonate (5.41 g) was obtained.

### Preparation Example 4

A mixture of 1-(2,3-dimethoxyphenyl)-N,N-dimethylmethanamine (3.00 g), sodium carbonate (1.63 g), sodium 2-bromoethane-1-sulfonate (3.24 g), water (6 mL), and ethanol (30 mL) was stirred at 75°C for 3 days. Sodium 2-bromoethane-1-sulfonate (3.24 g) was further added, and the mixture was stirred at 80°C for 2 days. Sodium 2-bromoethane-1-sulfonate (3.24 g) was further added, and the mixture was stirred at 80°C for 2 days. After allowing the mixture to cool to room temperature, concentration was performed under reduced pressure. Water was added to perform purification by reverse phase column chromatography (developing solvent: acetonitrile-water), and thereby 2-{[(2,3-dimethoxyphenyl)methyl](dimethyl)azaniumyl}ethane-1-sulfonate (3.50 g) was obtained.

### Preparation Example 5

A mixture of 1-(2,3-dimethoxyphenyl)-N,N-dimethylmethanamine (2.00 g), 1,2λ⁶-oxathiane-2,2-dione (1.36 mL), and ethyl acetate (20 mL) was stirred at 50°C for 3 hours, and then stirred at 70°C for 24 hours. 1,2λ⁶-oxathiane-2,2-dione (1.04 mL) was further added, and the mixture was stirred at 70°C for 24 hours. After allowing the mixture to cool to room temperature, the solid was collected by filtration, cleaned with ethyl acetate, and dried under reduced pressure. Thereby, 4-{[(2,3-dimethoxyphenyl)methyl](dimethyl)azaniumyl}butane-1-sulfonate (2.28 g) was obtained.

### Preparation Example 6

A mixture of 2-fluoro-4,5-dimethoxyaniline (2.50 g), 1,2λ⁶-oxathiolane-2,2-dione (1.54 mL) and acetonitrile (63 mL) was stirred at 115°C for 8 hours. 1,2λ⁶-oxathiolane-2,2-dione (0.64 mL) was further added, and the mixture was stirred at 115°C for 8 hours. After allowing the mixture to cool to room temperature, the solid was collected by filtration, cleaned with acetonitrile, and dried under reduced pressure at 50°C. Thereby, 3-(2-fluoro-4,5-dimethoxyanilino)propane-1-sulfonic acid (4.00 g) was obtained.

### Preparation Example 7

3,4-dimethoxyaniline (1.66 g), potassium iodide (1.79 g), and potassium carbonate (2.49 g) were added to a mixture of 3-(2-chloroethoxy)propane-1-sulfonic acid (1.46 g), dioxane (22 mL), and water (11 mL), and the mixture was stirred at 100°C overnight. The reaction liquid was allowed to cool to room temperature, and then concentrated, purified by reverse phase column chromatography (developing solvent: acetonitrile-water), and freeze-dried. Thereby, 3-[2-(3,4-dimethoxyanilino)ethoxy]propane-1-sulfonic acid (532 mg) was obtained.

### Preparation Example 8

A mixture of 2-methoxy-N-(2-methoxyethyl)ethan-1-amine (3.0 mL), 1,2λ⁶-oxathiolane-2,2-dione (2.0 mL), and acetonitrile (27 mL) was stirred at 80°C for 4 hours. The mixture was allowed to cool to room temperature, and then concentrated. Diethyl ether was added to the concentrate, and stirred at room temperature for 2 hours. Thereafter, the solid was collected by filtration, and dried under reduced pressure at room temperature. Thereby, 3-[bis(2-methoxyethyl)amino]propane-1-sulfonic acid (5.00 g) was obtained.

### Preparation Example 9

A mixture of 1-(2,3-dimethoxyphenyl)-N,N-dimethylmethanamine (1.70 g), sodium 3-chloro-2-hydroxypropane-1-sulfonate (3.42 g), potassium iodide (1.73 g), ethanol (26 mL), and water (7.7 mL) was stirred at 80°C overnight. The mixture was allowed to cool to room temperature, and then concentrated, purified by reverse phase column chromatography (developing solvent: acetonitrile-water), and freeze-dried. Thereby, 3-{[(2,3-dimethoxyphenyl)methyl](dimethyl)azaniumyl}-2-hydroxypropane-1-sulfonate (2.17 g) was obtained.

### Preparation Example 10

A mixture of 7,8-dimethoxy-1,2,3,4-tetrahydroisoquinoline (1.80 g), 1,2λ⁶-oxathiolane-2,2-dione (0.98 mL), potassium carbonate (1.29 g), and acetonitrile (45 mL) was stirred at 100°C for 8 hours. After allowing the mixture to cool to room temperature, water was added thereto. The mixture was concentrated, purified by reverse phase column chromatography (developing solvent: acetonitrile-water), and freeze-dried. Thereby, 3-(7,8-dimethoxy-3,4-dihydroisoquinolin-2(1H)-yl)propane-1-sulfonic acid (1.79 g) was obtained.

### Preparation Example 11

A mixture of 1-(2,3-dimethoxyphenyl)-N,N-dimethylmethanamine (1.30 g), diethyl(3-bromopropyl)phosphonate (1.66 mL), and ethanol (6.50 mL) was stirred at 80°C for 6 hours. The mixture was allowed to cool to room temperature, and then concentrated, and purified by reverse phase column chromatography (developing solvent: acetonitrile-water). Thereby, 3-(diethoxyphosphoryl)-N-[(2,3-dimethoxyphenyl)methyl]-N,N-dimethylpropan-1-aminium bromide (2.70 g) was obtained.

### Preparation Example 12

A mixture of 3-[bis(2-methoxyethyl)amino]propane-1-sulfonic acid (3.00 g), 1-(chloromethyl)-2,3-dimethoxybenzene (4.39 g), potassium carbonate (1.95 g), and ethanol (45 mL) was stirred at 80°C overnight. The mixture was allowed to cool to room temperature, and then concentrated, purified by reverse phase column chromatography (developing solvent: acetonitrile-water), and freeze-dried. Thereby, 3-{[(2,3-dimethoxyphenyl)methyl]bis(2-methoxyethyl)azaniumyl}propane-1-sulfonate (3.09 g) was obtained.

### Preparation Example 13

A mixture of diethyl(3-bromopropyl)phosphonate (2.53 g) and 3,4-dimethoxyaniline (3.00 g) was stirred at 95°C for 6 hours under an argon atmosphere. The mixture was allowed to cool to room temperature, a saturated aqueous solution of sodium hydrogen carbonate was added thereto, and the mixture was extracted once with ethyl acetate. The organic layer was cleaned once with saturated brine and dried over anhydrous magnesium sulfate. After filtration, concentration and purification by silica gel column chromatography (developing solvent: hexane-ethyl acetate, and then ethyl acetate-methanol) were performed. Thereby, diethyl[3-(3,4-dimethoxyanilino)propyl]phosphonate (1. 74 g) was obtained.

### Preparation Example 14

A mixture of 1-(2,3-dimethoxyphenyl)-N,N-dimethylmethanamine (2.00 g) and ethyl 4-bromobutanoate (2.60 g) was stirred at 80°C for 3 hours. This mixture was purified by reverse phase column chromatography (developing solvent: water-acetonitrile), and thereby N-[(2,3-dimethoxyphenyl)methyl]-4-ethoxy-N,N-dimethyl-4-oxobutan-1-aminium bromide (3.93 g) was obtained.

### Preparation Example 15

A mixture of 1-(6-fluoro-2,3-dimethoxyphenyl)-N,N-dimethylmethanamine (1.20 g), 1,2λ⁶-oxathiolane-2,2-dione (990 µL), and ethyl acetate (12 mL) was stirred at 50°C for 18 hours. After allowing the mixture to cool to room temperature, the solid was collected by filtration, cleaned with ethyl acetate, and dried under reduced pressure. Thereby, 3-{[(6-fluoro-2,3-dimethoxyphenyl)methyl](dimethyl)azaniumyl}propane-1-sulfonate (1.79 g) was obtained.

### Preparation Example 16

A mixture of 1-(2,3-dimethoxyphenyl)-N,N-dimethylmethanamine (2.00 g) and ethyl 5-bromopentanoate (2.79 g) was stirred at 80°C for 3 hours. This mixture was purified by reverse phase column chromatography (developing solvent: water-acetonitrile), and thereby N-[(2,3-dimethoxyphenyl)methyl]-5-ethoxy-N,N-dimethyl-5-oxopentan-1-aminium bromide (3.91 g) was obtained.

### Preparation Example 17

A mixture of 3-(2-fluoro-4,5-dimethoxyanilino)propane-1-sulfonic acid (4.00 g), potassium carbonate (4.52 g), methyl iodide (7.7 mL), and methanol (60 mL) was stirred at 50°C overnight. The mixture was allowed to cool to room temperature, and then concentrated, purified by reverse phase column chromatography (developing solvent: acetonitrile-water), and freeze-dried. Thereby, 3-[(2-fluoro-4,5-dimethoxyphenyl)(dimethyl)azaniumyl]propane-1-sulfonate (4.34 g) was obtained.

### Preparation Example 18

A mixture of 3-(3,4-dimethoxyanilino)propane-1-sulfonic acid (2.00 g), 1,4-diiodobutane (1.04 mL), potassium carbonate (2.21 g), dioxane (30 mL), and water (15 mL) was stirred at 100°C overnight. The mixture was allowed to cool to room temperature, and then concentrated, purified by reverse phase column chromatography (developing solvent: acetonitrile-water), and freeze-dried. Thereby, 3-[1-(3,4-dimethoxyphenyl)pyrrolidin-1-ium-1-yl]propane-1-sulfonate (2.37 g) was obtained.

### Preparation Example 19

A mixture of 3-(3,4-dimethoxyanilino)propane-1-sulfonic acid (2.00 g), ethyl iodide (2.94 mL), potassium carbonate (2.41 g), and methanol (30 mL) was stirred at 50°C overnight. Methyl iodide (4.1 mL) was added, and the mixture was subsequently stirred at 50°C overnight. The mixture was allowed to cool to room temperature, and then concentrated, purified by reverse phase column chromatography (developing solvent: acetonitrile-water), and freeze-dried. Thereby, 3-[(3,4-dimethoxyphenyl)(ethyl)(methyl)azaniumyl]propane-1-sulfonate (2.14 g) was obtained.

### Preparation Example 20

A mixture of 3-{[(2,3-dimethoxyphenyl)methyl](dimethyl)azaniumyl}propane-1-sulfonate (5.41 g) and 57% hydriodic acid (24 mL) was stirred at 110°C for 15 hours. After allowing the mixture to cool to room temperature, water (30 mL) was added, and concentration was performed under reduced pressure. This operation was repeated once again. Water (6 mL) was added to this concentrate to dissolve it. Thereafter, acetone (100 mL) was added, and the mixture was stirred in an ice bath for 3 minutes. The mixture was left to stand, and the supernatant was removed by decantation. Furthermore, water (6 mL) and acetone (75 mL) were added, and the same operation was performed again. Water (6 mL) and acetone (75 mL) were added to the resultant product, and the mixture was stirred in an ice bath for 3 minutes. Thereafter, the solid was collected by filtration, cleaned with acetone, dried under reduced pressure. Thereby, 3-{[(2,3-dihydroxyphenyl)methyl](dimethyl)azaniumyl}propane-1-sulfonate (5.02 g) was obtained.

### Preparation Example 21

Under an argon atmosphere, and under cooling in a dry ice-acetone bath, a 1 mol/L tribromoborane dichloromethane solution (19.2 mL) was added dropwise to a mixture of 3-{[(2,3-dimethoxyphenyl)methyl]bis(2-methoxyethyl)azaniumyl}propane-1-sulfonate (2.59 g) and dichloromethane (52 mL). The temperature was slowly raised to room temperature over 3 hours, and the mixture was stirred at room temperature for 2 hours. Methanol was added under ice-cooling, and the mixture was stirred at room temperature for 30 minutes and concentrated under reduced pressure. Methanol was added to the residue, and the resultant mixture was concentrated under reduced pressure again. This operation was performed twice later, and concentration, purification by reverse phase column chromatography (developing solvent: acetonitrile-water), and freeze-drying were performed. Thereby, 3-{[(2,3-dihydroxyphenyl)methyl]bis(2-methoxyethyl)azaniumyl}propane-1-sulfonate (674 mg) was obtained.

### Preparation Example 22

A mixture of 4-{[(2,3-dimethoxyphenyl)methyl](dimethyl)azaniumyl}butane-1-sulfonate (2.28 g) and 57% hydriodic acid (9.6 mL) was stirred at 110°C for 4 hours. After allowing the mixture to cool to room temperature, water was added, and concentration was performed under reduced pressure. This operation was repeated once again. Water (4 mL) was added to this concentrate to dissolve it. Thereafter, acetone (80 mL) was added, and the mixture was stirred and allowed to stand. Thereafter, the supernatant was removed by decantation. Furthermore, Millipore water (4 mL) and acetone (60 mL) were added, and the same operation was performed. Millipore water (4 mL) and acetone (60 mL) were added to the resultant product, and the mixture was stirred. Thereafter, the solid was collected by filtration, cleaned with acetone, dried under reduced pressure. Thereby, 4-{[(2,3-dihydroxyphenyl)methyl](dimethyl)azaniumyl}butane-1-sulfonate (2.45 g) was obtained.

### Preparation Example 23

A mixture of 3-{[(6-fluoro-2,3-dimethoxyphenyl)methyl](dimethyl)azaniumyl}propane-1-sulfonate (1.79 g) and 57% hydriodic acid (7.5 mL) was stirred at 110°C for 6 hours. After allowing the mixture to cool to room temperature, water was added, and concentration was performed under reduced pressure. This operation was repeated once again. Acetone (70 mL) was added to the resultant product, and the mixture was stirred under ice-cooling. The mixture was left to stand overnight to precipitate a solid, and then the mixture was stirred under ice-cooling for 1 hour. The mixture was left to stand, and the supernatant was removed by decantation. Acetone was added to this resultant product. Thereafter, the solid was collected by filtration, cleaned with acetone, and dried under reduced pressure. Thereby, 3-{[(6-fluoro-2,3-dihydroxyphenyl)methyl](dimethyl)azaniumyl}propane-1-sulfonate (1.58 g) was obtained.

### Preparation Example 24

A mixture of 3-(diethoxyphosphoryl)-N-[(2,3-dimethoxyphenyl)methyl]-N,N-dimethylpropan-1-aminium bromide (2.80 g) and 57% hydriodic acid (8 mL) was stirred at 100°C for 18 hours. After allowing the mixture to cool to room temperature, water and acetone were added, and concentration was performed under reduced pressure. Water was added to this concentrate, and concentration was performed under reduced pressure. Water was added to this concentrate, an insoluble material was separated by filtration, and the filtrate was concentrated under reduced pressure. Acetone was added to this concentrate, and the resulting solid was filtered. This filtered product was cleaned with acetone and dried under reduced pressure, and thereby N-[(2,3-dihydroxyphenyl)methyl]-N,N-dimethyl-3-phosphonopropan-1-aminium iodide (571 mg) was obtained.

### Preparation Example 25

A mixture of N-[(2,3-dimethoxyphenyl)methyl]-4-ethoxy-N,N-dimethyl-4-oxobutan-1-aminium bromide (3.91 g) and 57% hydriodic acid (22.5 g) was stirred at 110°C for 15 hours. The mixture was concentrated, water was added to the obtained residue, and concentration was performed under reduced pressure. This operation was repeated once more. Acetone was added to the resultant product, and cooling was performed in an ice bath to remove the supernatant. Acetone was added to resultant product, cooling was performed in an ice bath, and the resulting solid was filtered. This filtered product was cleaned with acetone, and thereby 3-carboxy-N-[(2,3-dihydroxyphenyl)methyl]-N,N-dimethylpropan-1-aminium iodide (2.13 g) was obtained.

### Preparation Example 26

A mixture of N-[(2,3-dimethoxyphenyl)methyl]-5-ethoxy-N,N-dimethyl-5-oxopentan-1-aminium bromide (3.90 g) and 57% hydriodic acid (22.0 g) was stirred at 110°C for 16 hours. The mixture was concentrated, water was added to the obtained residue, and concentration was performed under reduced pressure. This operation was repeated once more. Acetone was added to the resultant product, and cooling was performed in an ice bath to remove the supernatant. Acetone was added to resultant product, cooling was performed in an ice bath, and the resulting solid was filtered. This filtered product was cleaned with acetone, and thereby 4-carboxy-N-[(2,3-dihydroxyphenyl)methyl]-N,N-dimethylbutan-1-aminium iodide (1.33 g) was obtained. All the filtrates were concentrated, and the obtained residue was purified by reverse phase column chromatography (developing solvent: water-acetonitrile). Acetone was added to the concentrated solid to perform filtration. This filtered product was cleaned with acetone, and thereby 4-carboxy-N-[(2,3-dihydroxyphenyl)methyl]-N,N-dimethylbutan-1-aminium iodide (1.29 g) was obtained.

### Preparation Example 27

Iron(III) chloride hexahydrate (5.80 g), sodium oleate (19.5 g), ethanol (43 mL), water (33 mL), and hexane (75 mL) were mixed and heated to reflux for 4 hours at 70°C under an argon atmosphere. The mixture was allowed to cool, and was transferred to a liquid separation funnel to remove the aqueous layer. 50 mL of water was added for cleaning, and the organic layer was recovered. This operation was further repeated for two times (using 50% methanol water in the second time). The obtained organic layer was dried over sodium sulfate, and concentrated under reduced pressure to obtain an iron oleate complex (FeOA₃, 19.2 g).

### Preparation Example 28

A mixture of FeOA₃ (6.53 g), oleyl alcohol (11.7 g), and diphenyl ether (36.4 g) was stirred under reduced pressure at 90°C for 2 hours. Thereafter, the pressure was returned to normal pressure with argon, the temperature was raised to a bath temperature of 213°C over 16 minutes, and stirring was performed for 30 minutes after the internal temperature exceeded 200°C. After allowing the resultant product to cool to room temperature, hexane (5 mL) and acetone (150 mL) were added. A resultant mixture was centrifuged at 8,000 rpm for 10 minutes at 10°C to remove the supernatant. Hexane (24 mL) was added to the obtained precipitate, and acetone (150 mL) was further added. Thereafter, the centrifugal separation was performed at 10°C and 8,000 rpm for 10 minutes to remove the supernatant. This operation was repeated once more, and the obtained precipitate was dried under reduced pressure. Thereby, iron oxide nanoparticles (SNP-OA, 992 mg) having a surface to which oleic acid was coordination bonded were obtained.

### Preparation Example 33

Sodium carbonate (15.6 g) and sodium 2-bromoethane-1-sulfonate (23.3 g) were added to a mixture of 2-(2,3-dimethoxyphenyl)-N, N-dimethylethan-1-amine (7.71 g), water (15.4 mL), and ethanol (77 mL), and the mixture was stirred at 80°C for 18 hours. Sodium 2-bromoethane-1-sulfonate (11.7 g), sodium carbonate (7.81 g), ethanol (20 mL), and water (4 mL) were added, and the mixture was stirred at 80°C for 1 day. After concentration, purification by reverse phase column chromatography (developing solvent: water-acetonitrile) was performed, and thereby 2-{[2-(2,3-dimethoxyphenyl)ethyl](dimethyl)azaniumyl}ethane-1-sulfonate (9.00 g) was obtained.

### Preparation Example 35

A mixture of 2,3-dimethoxyaniline (5.61 g), 1,2λ⁶-oxathiolane-2,2-dione (5.83 g), and acetonitrile (140 mL) was refluxed for 8 hours. The resultant product was allowed to cool to room temperature, and stirred in an ice bath. The solid was collected by filtration, and cleaned with cold acetonitrile. Thereby, 3-(2,3-dimethoxyanilino)propane-1-sulfonic acid (5.59 g) was obtained.

### Preparation Example 38

A mixture of 3-(2,3-dimethoxyanilino)propane-1-sulfonic acid (5.58 g), potassium carbonate (6.72 g), methyl iodide (11.4 mL), and methanol (85 mL) was stirred at 50°C for 8 hours. Methyl iodide (11.4 mL) was added, and the mixture was stirred at 50°C for 24 hours. After filtration of insoluble material, concentration, and purification with Sepabeads (registered trademark) SP207SS were performed. Concentration was performed, and the obtained solid was dissolved in ethanol under heating. The resultant product was allowed to cool to room temperature, and then stirred in an ice bath. The solid was filtered, and cleaned with cold ethanol. Thereby, 3-[(2,3-dimethoxyphenyl)(dimethyl)azaniumyl]propane-1-sulfonate (5.40 g) was obtained.

### Preparation Example 43

A mixture of 3-[(2,3-dimethoxyphenyl)(dimethyl)azaniumyl]propane-1-sulfonate (5.40 g) and 57% hydriodic acid (40 g) was refluxed for 8 hours. After allowing the mixture to cool to room temperature, water was added, and concentration was performed under reduced pressure. This operation was repeated twice more. Water (3 mL) was added to this concentrate to dissolve it. Thereafter, acetone (50 mL) was added, and the mixture was stirred in an ice bath for 30 minutes. The mixture was left to stand, and the supernatant was removed by decantation. Furthermore, water (3 mL) and acetone (40 mL) were added, and the same operation was performed again. Water (3 mL) and acetone (40 mL) were added to the resultant product, and the mixture was stirred in an ice bath for 30 minutes. The solid was filtered, and cleaned with acetone. Thereby, 3-[(2,3-dihydroxyphenyl)(dimethyl)azaniumyl]propane-1-sulfonate (4.35 g) was obtained.

### Preparation Example 50

A mixture of 2-{[2-(2,3-dimethoxyphenyl)ethyl](dimethyl)azaniumyl}ethane-1-sulfonate (9.00 g) and 57% hydriodic acid (40 mL) was stirred at 100°C for 15 hours. The mixture was concentrated, acetone was added. The mixture was stirred in an ice bath for 5 minutes. The resulting solid was filtered, and cleaned with acetone. Thereby, 2-{[2-(2,3-dihydroxyphenyl)ethyl](dimethyl)azaniumyl}ethane-1-sulfonate (3.40 g) was obtained.

### Preparation Example 58

A mixture of methyl(piperidin-4-yl)acetate monohydrochloride (5.00 g), 1-(chloromethyl)-2,3-dimethoxybenzene (5.78 g), potassium carbonate (4.64 g), and acetonitrile (50 mL) was stirred at room temperature overnight. The reaction mixture was filtered, the filtrate was concentrated, and purification was performed by basic silica gel column chromatography (developing solvent: hexane-ethyl acetate). Thereby, methyl {1-[(2,3-dimethoxyphenyl)methyl]piperidin-4-yl}acetate (4.90 g) was obtained.

### Preparation Example 59

A mixture of 2-(2,3-dimethoxyphenyl)-N,N-dimethylethan-1-amine (10.9 g) and ethyl 4-bromobutanoate (8.28 mL) was stirred at 80°C for 3 hours. This mixture was purified by reverse phase column chromatography (developing solvent: water-acetonitrile), and thereby N-[2-(2,3-dimethoxyphenyl)ethyl]-4-ethoxy-N,N-dimethyl-4-oxobutan-1-aminium bromide (15.7 g) was obtained.

### Preparation Example 60

A mixture of 2,3-dimethoxyaniline (5.00 g) and 1,2λ⁶-oxathiane-2,2-dione (5.78 g) was stirred at 95°C for 24 hours. This mixture was purified by reverse phase column chromatography (developing solvent: acetonitrile-water). Concentration was performed, and the obtained solid was cleaned with acetonitrile. Thereby, 4-(2,3-dimethoxyanilino)butane-1-sulfonic acid (4.78 g) was obtained.

### Preparation Example 61

A mixture of 2,3-dimethoxyaniline (5.00 g), ethyl 5-bromopentanoate (8.19 g), and triethylamine (3.96 g) was stirred at room temperature for 5 days. Water was added, and the mixture was extracted once with ethyl acetate. The organic layer was cleaned once with saturated brine and dried over anhydrous magnesium sulfate. After filtration, concentration and purification by silica gel column chromatography (developing solvent: hexane-ethyl acetate in the first time, and chloroform-ethyl acetate in the second time) were performed. Thereby, ethyl 5-(2,3-dimethoxyanilino)pentanoate (6.26 g) was obtained.

### Preparation Example 62

A mixture of methyl {1-[(2,3-dimethoxyphenyl)methyl]piperidin-4-yl}acetate (4.90 g), methyl iodide (5.0 mL), and methanol (74 mL) was stirred at 50°C for 4 hours. The mixture was allowed to cool to room temperature, concentrated, and purified by reverse phase silica gel column chromatography (developing solvent: water-acetonitrile). Thereby, 1-[(2,3-dimethoxyphenyl)methyl]-4-(2-methoxy-2-oxoethyl)-1-methylpiperidin-1-ium iodide (6.54 g) was obtained.

### Preparation Example 63

A mixture of ethyl 1-[(2,3-dimethoxyphenyl)methyl]piperidine-4-carboxylate (18.8 g), methyl iodide (19.1 mL), and ethanol (188 mL) was stirred at 50°C for 4 hours. The mixture was allowed to cool to room temperature, concentrated, and purified by reverse phase silica gel column chromatography (developing solvent: water-acetonitrile). Thereby, 1-[(2,3-dimethoxyphenyl)methyl]-4-(ethoxycarbonyl)-1-methylpiperidin-1-ium iodide (25.9 g) was obtained.

### Preparation Example 64

A mixture of 1-[(2,3-dimethoxyphenyl)methyl]-4-(2-methoxy-2-oxoethy1)-1-methylpiperidin-1-ium iodide (6.54 g) and 57% hydriodic acid (19 mL) was stirred at 100°C for 6 hours. After allowing the mixture to cool to room temperature, water was added, and concentration was performed under reduced pressure. This operation was repeated twice more. Acetone (30 mL) was added to the resultant product. The mixture was stirred at room temperature, and then cooled in an ice bath, and left to stand. The supernatant was removed by decantation. Acetone was further added, and the same operation was performed twice more. Acetone (30 mL) was added to the resultant product. The mixture was stirred at room temperature, and then cooled in an ice bath. The solid was collected by filtration, and thereby 4-(carboxymethyl)-1-[(2,3-dihydroxyphenyl)methyl]-1-methylpiperidin-1-ium iodide (4.49 g) was obtained.

### Preparation Example 65

A mixture of 1-[(2,3-dimethoxyphenyl)methyl]-4-(ethoxycarbonyl)-1-methylpiperidin-1-ium iodide (25.9 g) and 57% hydriodic acid (76 mL) was stirred overnight at 100°C. After allowing the mixture to cool to room temperature, water was added, and concentration was performed under reduced pressure. This operation was repeated twice more. Acetone was added to the resultant product. The mixture was stirred at room temperature, and then cooled in an ice bath, and left to stand. The supernatant was removed by decantation. Acetone was further added, and the same operation was performed once more. Acetone was added to the resultant product. The mixture was stirred at room temperature, and then cooled in an ice bath. The solid was collected by filtration, and thereby 4-carboxy-1-[(2,3-dihydroxyphenyl)methyl]-1-methylpiperidin-1-ium iodide (10.8 g) was obtained. Furthermore, the filtrate was concentrated, and purified by reverse phase silica gel column chromatography (developing solvent: water-acetonitrile). Thereby, 4-carboxy-1-[(2,3-dihydroxyphenyl)methyl]-1-methylpiperidin-1-ium iodide (12.0 g) was obtained.

### Preparation Example 66

A mixture of N-[2-(2,3-dimethoxyphenyl)ethyl]-4-ethoxy-N,N-dimethyl-4-oxobutan-1-aminium bromide (15.7 g) and 57% hydriodic acid (52 mL) was stirred at 100°C for 18 hours. The mixture was concentrated, water was added to the obtained residue, and concentration was performed under reduced pressure. Acetonitrile was added to the resultant product. Cooling in an ice bath was performed. A solid was precipitated, and then concentrated. Acetone was added to the resultant product, and the mixture was stirred at room temperature for 10 minutes. Thereafter, the solid was filtered, and thereby 3-carboxy-N-[2-(2,3-dihydroxyphenyl)ethyl]-N,N-dimethylpropan-1-aminium iodide (14.8 g) was obtained.

### Example 1

A reaction scheme is shown in Fig. 1.

Oleyl alcohol (25.6 mL) and Ph₂O (20 mL) were added into a 200 mL three neck flask, and molecular sieves 3A (5 g) were further added as a desiccant. Thereafter, the temperature was raised to an internal temperature of 170°C (first solution). The heptane solution of the iron oleate complex obtained in Reference Example 3 (equivalent to 10 g as the iron oleate complex), and Ph₂O (10 mL) were added into another 200 mL three neck flask, and heated to an external temperature of 90°C. The mixture was stirred under reduced pressure for about 2 hours (second solution). In a nitrogen atmosphere and for about 1 hour, the second solution was added dropwise into the first solution heated to an internal temperature of 170°C while maintaining its internal temperature. After completion of the dropwise addition, the reaction was continued for 3 hours at the same internal temperature, and thereafter cooling to room temperature was performed. The cooled slurry was filtered to remove molecular sieves, and washed with heptane (33 mL). Acetone (156 mL) was added to the obtained filtrate and a filtrate wash liquid containing a wash liquid, and thereafter centrifugal separation (7,000 rpm, 10 minutes, 10°C) was performed. The supernatant liquid was removed, the centrifuge tube residue was dissolved in heptane (20 mL), and acetone (63 mL) was added. Thereafter, centrifugal separation (7,000 rpm, 10 minutes, 10°C) was performed twice. The supernatant liquid was removed, and thereafter the resultant product was transferred to a 100 mL round-bottom flask using heptane. Thereafter, concentration was performed at a bath temperature of 30°C. Furthermore, drying under reduced pressure was performed, and thereby SNP-OA (1.50 g, weight yield 15.0% (w/w: weight percentage)) was obtained.

### Example 2

The slurry obtained in the same manner as in Example 1 was filtered. Heptane, acetone, and hexane were added to the filtrate wash liquid from which molecular sieves were removed to dilute the slurry. Thereafter, the resultant product was circulated by a ceramic membrane filtration device and concentrated (pressurized with air, 0.5 MPa). After concentration, washing was performed with a mixed solution of heptane/acetone = 3/1. After confirming that the washing liquid was no longer colored, the residue (retentate) was recovered using heptane. The obtained recovered solution was concentrated, and dried under reduced pressure. Thereby, SNP-OA (0.73 g using the equivalent of 5 g of an iron oleate complex, weight yield 14.6% (w/w)) was obtained.

### Example 3

The heptane solution of the iron oleate complex (equivalent to 500 g as an iron oleate complex) obtained in the same manner as in Reference Example 3 was added to Ph₂O (500 mL), heated to 50°C, and stirred under reduced pressure for about 4.5 hours. Thereafter, the mixture was further heated to 90°C, and stirred under reduced pressure for 18 hours. Thereby, a Ph₂O solution (1001.48 g) of the iron oleate complex was obtained. 20.04 g (equivalent to 10 g of the iron oleate complex) was extracted from the prepared Ph₂O solution of the iron oleate complex, and oleic acid (6.28 g) was added to the extracted solution (second solution). Oleyl alcohol (21.8 g), Ph₂O (20 mL), and molecular sieves 3A (5 g) were added to a 200 mL three neck flask, and the temperature was raised to an internal temperature of 170°C (first solution). For about 1 hour, the second solution was added dropwise into the first solution heated to an internal temperature of 170°C while maintaining its internal temperature. After completion of the dropwise addition, the reaction was continued for 3 hours at the same internal temperature, and thereafter cooling to room temperature was performed. The cooled slurry was filtered to remove molecular sieves, and washed with heptane (33 mL). Acetone (156 mL) was added to the combination of the obtained filtrate and a wash liquid, and thereafter centrifugal separation (7,000 rpm, 10 minutes, 10°C) was performed. The supernatant liquid was removed, the centrifuge tube residue was dissolved in heptane (20 mL), and acetone (63 mL) was added. Thereafter, centrifugal separation (7,000 rpm, 10 minutes, 10°C) was performed twice. The supernatant liquid was removed, and thereafter the resultant product was transferred to a 100 mL round-bottom flask using heptane. Thereafter, concentration was performed at room temperature. Furthermore, drying under reduced pressure was performed, and thereby SNP-OA (1.47 g, weight yield 14.7% (w/w)) was obtained.

### Example 4

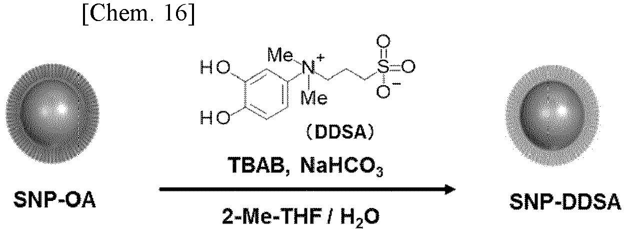

DDSA (4 g) was added to water (33 mL) and stirred. Substitution with nitrogen was performed, and thereby a slurry was prepared. The temperature was raised to 50°C or higher for dissolution. After confirming the dissolution, sodium hydrogen carbonate (1.04 g) was added at 56°C, and cooling to around room temperature was performed. After cooling, a 2-Me-THF (15 mL) dispersion liquid of SNP-OA (1 g) obtained in Example 1 was added under a nitrogen atmosphere, after confirming that a pH was about 7.5. Furthermore, TBAB (1.32 g) was added, and the temperature was raised to 40°C to 50°C. The reaction was carried out at the same temperature for 6 hours. The reaction liquid was cooled with water, heptane (20 mL) was added, and the mixture was stirred. Thereafter, while washing the mixture with water (5 mL), the mixture was transferred to a separatory funnel for liquid separation. Heptane (20 mL) was added to the obtained aqueous layer and washed. The obtained aqueous layer was quantified by size exclusion chromatography (38.8% (w/w) as target SNP-DDSA). While washing using water, filtration was performed through a 10K membrane (Hydrosart (registered trademark) 10K, 0.02m²) using a TFF device, and the residue (retentate) was recovered. Subsequently, filtration was performed through a 300K membrane (Pellicon (registered trademark) XL Cassette Ultracel 300K, 0.005m²), washing was performed with water, and the filtrate was recovered. Thereby, an aqueous dispersion liquid equivalent to 329 mg (weight yield 32.9% (w/w)) as the target SNP-DDSA was obtained.

As a result of observing the obtained SNP-DDSA dispersion liquid by TEM, it was estimated that an average particle diameter of the SNP, which was the core, in the obtained SNP-DDSA was 3.6 nm from an average value of particle diameters of 100 SNPs.

### Example 5

DDSA (807 mg) and water (6.7 mL) were added to a 30 mL three neck flask under an argon atmosphere, and the mixture was heated and dissolved. Sodium hydrogen carbonate (204 mg) was added to the obtained solution, and the pH was adjusted to 6.86. TBAF trihydrate (151 mg) was further added. The obtained aqueous solution was added to a chloroform (6 mL) dispersion liquid of SNP-OA (120 mg), and then the temperature was raised to an internal temperature of 50°C. After stirring at the same temperature for 1.5 hours, the layers were separated when the temperature was cooled to room temperature. After extracting the upper aqueous layer, extraction was performed three times using a small amount of water. The combined aqueous layer was filtered with PBS 3 times and finally with water one time through an Amicon (registered trademark) cut off filter (100K) (6,000 rpm, 15 to 40 minutes). All the obtained filtrates were put through the Amicon (registered trademark) cut off filter (10K) and filtered. The filtrate became substantially transparent by filtration 8 times using this filter. The residue (retentate) on the 10K filter membrane was recovered and freeze-dried. Thereby, SNP-DDSA (54.4 mg, weight yield 45.3% (w/w)) was obtained.

### Example 6

Water (36.3 mL) was added to DDSA (4.39 g) and stirred to prepare a slurry. After raising the temperature to about 45°C, sodium hydrogen carbonate (1.14 g) was added at the same temperature, and dissolution was confirmed. TBAB (1.45 g) was further added to the solution at a pH about 7.4. After adding SNP-OA (content 1.10 g), which is a heptane solution, the reaction was started by washing with 1-butanol (16.5 mL). After reacting at 40°C to 45°C for 5 hours, the reaction mixture was cooled to room temperature, heptane (33 mL) was added, and the mixture was stirred. The solution was separated to obtain an aqueous layer. Subsequently, water (4.4 mL) was added to the heptane layer for washing. Thereafter, liquid separation was performed. The obtained aqueous layer was combined with the previously obtained aqueous layer (48.95 g), and quantified by size exclusion chromatography (29.5% (w/w) as target SNP-DDSA). Purification with a 10K membrane and a 300K membrane was performed using a stirring type cell, which is a TFF device. Thereby, an aqueous dispersion liquid equivalent to 298 mg (weight yield 27.1% (w/w)) of the target SNP-DDSA was obtained.

As a result of observing the obtained SNP-DDSA dispersion liquid by TEM, it was estimated that an average particle diameter of the SNP, which was the core, of the obtained SNP-DDSA was 3.2 nm from an average value of particle diameters of 100 SNPs.

### Example 7-1

Sodium hydrogen carbonate (34 mg) was added to a mixture of 4-{[(2,3-dihydroxyphenyl)methyl](dimethyl)azaniumyl}butane-1-sulfonate (275 mg) and water (2.2 mL). This solution was added to a mixture of SNP-OA (20mg) and chloroform (2.5 mL). Thereafter, a mixture of TBAF trihydrate (63 mg) and water (300 µL) was added, and the mixture was stirred at room temperature for 16 hours under an argon atmosphere. The aqueous layer was separated, and the chloroform layer was extracted twice with water. The aqueous layer was collected, dispersed in PBS, and transferred to an Amicon (registered trademark) 30K filter, and centrifugal separation was performed at 10°C and 5,800 rpm for 15 minutes. The obtained filtrate was subjected to centrifugal separation at 10°C and 5,800 rpm for 30 minutes using an Amicon (registered trademark) 10K filter. This series of operations was performed three more times. Water was added to the concentrated liquid on the Amicon (registered trademark) 30K filter, and the mixture was subjected to centrifugal separation at 10°C and 5,800 rpm for 15 minutes. The obtained filtrate was subjected to centrifugal separation at 10°C and 5,800 rpm for 30 minutes using an Amicon (registered trademark) 10K filter. This series of operations was performed twice more. Water was added to the concentrated liquid on the Amicon (registered trademark) 10K filter, and the mixture was subjected to centrifugal separation at 10°C and 5,800 rpm for 30 minutes. This operation was performed five more times. The concentrated liquid was filtered through a membrane (0.2 µm) and freeze-dried. Thereby, 10K purified particles (30.3 mg) were obtained. The filtrates washed with an Amicon (registered trademark) 10K filter were sequentially subjected to centrifugal separation at 10°C and 5,800 rpm for 60 minutes using the Amicon (registered trademark) 3K filter. Water was further added to the resultant product, and the mixture was subjected to centrifugal separation at 10°C and 5,800 rpm for 60 minutes. The concentrated liquid was filtered through a membrane (0.2 µm) and freeze-dried. Thereby, 3K purified particles (17.8 mg) were obtained.

### Example 7-2

3-{[(6-Fluoro-2,3-dihydroxyphenyl)methyl](dimethyl)azaniumyl}propane-1-sulfonate (280 mg) was dissolved in water (2.2 mL), and sodium hydrogen carbonate (38 mg) was added. This solution was added to a chloroform (2.5 mL) solution of SNP-OA (20mg), and a water (0.3 mL) solution of TBAF trihydrate (65 mg) was further added. This mixture was stirred under an argon atmosphere at room temperature for 20 hours. An insoluble material was filtered, and the aqueous layer was transferred to an Amicon (registered trademark) 30K filter and subjected to centrifugal separation at 10°C and 5,800 rpm for 30 minutes. PBS was added to the resultant product, and the mixture was subjected to centrifugal separation at 10°C and 5,800 rpm for 30 minutes. The filtrates obtained by first two washing operations with an Amicon (registered trademark) 30K filter were subjected to centrifugal separation at 10°C and 5,800 rpm for 30 minutes using the Amicon (registered trademark) 10K filter. Water was further added to the resultant product, and the mixture was subjected to centrifugal separation at 10°C and 5,800 rpm for 30 minutes. Water was further added to the resultant product, and the mixture was subjected to centrifugal separation at 10°C and 5,800 rpm for 60 minutes. This operation was performed 12 more times. The concentrated liquid was filtered through a membrane (0.2 µm) and freeze-dried, and thereby 10K purified particles (13.5 mg) were obtained. The filtrates obtained by first five washing operations with an Amicon (registered trademark) 10K filter were subjected to centrifugal separation at 10°C and 5,800 rpm for 30 to 60 minutes using the Amicon (registered trademark) 3K filter. Water was further added to the resultant product, and the mixture was subjected to centrifugal separation at 10°C and 5,800 rpm for 60 minutes. This operation was performed nine more times. The concentrated liquid was filtered through a membrane (0.2 µm) and freeze-dried, and thereby 3K purified particles (7.3 mg) were obtained.

### Example 7-3

3,4-Dihydroxy-N,N-dimethyl-N-(3-phosphonopropyl)anilinium iodide (265 mg) was dissolved in water (2.4 mL). Sodium hydrogen carbonate (100 mg) and a water (0.3 mL) solution of TBAF trihydrate (67 mg) were added into this aqueous solution. This solution was added to a chloroform (2.5 mL) solution of SNP-OA (20 mg), and rinsed with water (0.6 mL). This mixture was stirred under an argon atmosphere at room temperature for 18 hours. An insoluble material was filtered, and the aqueous layer was transferred to an Amicon (registered trademark) 100K filter and subjected to centrifugal separation at 10°C and 5,800 rpm for 15 minutes. The obtained filtrate was subjected to centrifugal separation at 10°C and 5,800 rpm for 30 minutes using an Amicon (registered trademark) 10K filter. Water was further added to the resultant product, and the mixture was subjected to centrifugal separation at 10°C and 5,800 rpm for 30 minutes. Water was further added to the resultant product, and the mixture was subjected to centrifugal separation at 10°C and 5,800 rpm for 60 minutes. This operation was performed eight more times. The concentrated liquid was filtered through a membrane (0.2 µm) and freeze-dried, and thereby 10K purified particles (1.0 mg) were obtained. The filtrates obtained by first two washing operations with an Amicon (registered trademark) 10K filter were subjected to centrifugal separation at 10°C and 5,800 rpm for 30 to 60 minutes using the Amicon (registered trademark) 3K filter. Water was further added to the resultant product, and the mixture was subjected to centrifugal separation at 10°C and 5,800 rpm for 60 minutes. This operation was performed seven more times. The concentrated liquid was filtered through a membrane (0.2 µm) and freeze-dried, and thereby 3K purified particles (1.4 mg) were obtained.

Structural formulas and physicochemical data of the nanoparticles obtained in Examples 7-1 to 7-3 are shown in the table below.

Furthermore, another nanoparticles, which are objective substances, can be produced by carrying out the phase transfer catalyst method of the present invention in the same manner as in the above-described examples using another zwitterionic ligands shown in the preparation example table to be described later.

### Comparative Example 1

### (First step)

A mixture of SNP-OA(100 mg), MEAA(2.5 mL), and methanol (7.5 mL) was stirred at 70°C for 5 hours under an argon atmosphere. After allowing the mixture to cool to room temperature, concentration was performed under reduced pressure. Acetone (24 mL) and hexane (96 mL) were added. The reaction mixture was divided into 6 parts, centrifugal separation was performed at 10°C and 7,000 rpm for 10 minutes to remove the supernatant. Thereby, SNP-MEAA was obtained.

### (Second step)

Sodium hydrogen carbonate (900 mg) was added to a mixture of 4-{[(2,3-dihydroxyphenyl)methyl](dimethyl)azaniumyl}butane-1-sulfonate (1.31 g) and water (40 mL). A DMF (8 mL) solution of the previously mentioned SNP-MEAA was added to the mixture, and the mixture was stirred at room temperature for 16 hours under an argon atmosphere. The reaction mixture was divided into 6 parts using water (3 mL), acetone (30 mL) was added to each of the parts, and centrifugal separation was performed at 10°C and 7,000 rpm for 10 minutes to remove the supernatant. The obtained precipitate was dispersed in PBS, and centrifugal separation was performed at 10°C and 5,800 rpm for 30 minutes using an Amicon (registered trademark) 30K filter. The obtained filtrate was subjected to centrifugal separation at 10°C and 5,800 rpm for 30 minutes using an Amicon (registered trademark) 10K filter. This series of operations was performed three more times. Water was added to the concentrated liquid on the Amicon (registered trademark) 30K filter, and the mixture was subjected to centrifugal separation at 10°C and 5,800 rpm for 30 minutes. The obtained filtrate was subjected to centrifugal separation at 10°C and 5,800 rpm for 30 minutes using an Amicon (registered trademark) 10K filter. This series of operations was performed six more times. Water was added to the concentrated liquid on the Amicon (registered trademark) 10K filter, and the mixture was subjected to centrifugal separation at 10°C and 5,800 rpm for 30 minutes. This operation was performed seven more times. The concentrated liquid was filtered through a membrane (0.2 µm) and freeze-dried. Thereby, 10K purified particles (117.7 mg) were obtained. The filtrates washed with an Amicon (registered trademark) 10K filter were sequentially subjected to centrifugal separation at 10°C and 5,800 rpm for 60 minutes using the Amicon (registered trademark) 3K filter. Water was further added to the resultant product, and the mixture was subjected to centrifugal separation at 10°C and 5,800 rpm for 60 minutes. The concentrated liquid was filtered through a membrane (0.2 µm) and freeze-dried. Thereby, 3K purified particles (53.2 mg) were obtained. The 3K purified particles were acid-hydrolyzed with hydrochloric acid, and analyzed by HPLC. As a result, the presence of zwitterionic ligands and 4-{[(2,3-dihydroxyphenyl)methyl](dimethyl)azaniumyl}butane-1-sulfonate was confirmed, and therefore it was confirmed that the zwitterionic ligands were coordination bonded to the 3K purified particles.
Conditions for HPLC are shown below.
Column: YMC Triart C18
Eluent: 10 mM dipotassium hydrogen phosphate (pH 6.0)/acetonitrile (98:2)

### Comparative Example 2

### (First step)

A mixture of SNP-OA(100 mg), MEAA(2.5 mL), and methanol (7.5 mL) was stirred at 70°C for 5 hours under an argon atmosphere. After allowing the mixture to cool to room temperature, concentration was performed under reduced pressure. Acetone (24 mL) and hexane (96 mL) were added. The reaction mixture was divided into 6 parts, centrifugal separation was performed at 10°C and 7,000 rpm for 10 minutes to remove the supernatant. Thereby, SNP-MEAA was obtained.

### (Second step)

Sodium hydrogen carbonate (650 mg) was added to a mixture of 3-{[(6-fluoro-2,3-dihydroxyphenyl)methyl](dimethyl)azaniumyl}propane-1-sulfonate (1.32 g) and water (40 mL). A DMF (8 mL) solution of the previously mentioned SNP-MEAA was added to the mixture, and the mixture was stirred at room temperature for 16 hours under an argon atmosphere. The reaction mixture was divided into 6 parts using water (3 mL), acetone (30 mL) was added to each of the parts, and centrifugal separation was performed at 10°C and 7,000 rpm for 10 minutes to remove the supernatant. The obtained precipitate was dispersed in PBS, and centrifugal separation was performed at 10°C and 5,800 rpm for 30 minutes using an Amicon (registered trademark) 30K filter. The obtained filtrate was subjected to centrifugal separation at 10°C and 5,800 rpm for 30 minutes using an Amicon (registered trademark) 10K filter. This series of operations was performed three more times. Water was added to the concentrated liquid on the Amicon (registered trademark) 30K filter, and the mixture was subjected to centrifugal separation at 10°C and 5,800 rpm for 30 minutes. The obtained filtrate was subjected to centrifugal separation at 10°C and 5,800 rpm for 30 minutes using an Amicon (registered trademark) 10K filter. This series of operations was performed seven more times. Water was added to the concentrated liquid on the Amicon (registered trademark) 10K filter, and the mixture was subjected to centrifugal separation at 10°C and 5,800 rpm for 30 minutes. This operation was performed four more times. The concentrated liquid was filtered through a membrane (0.2 and freeze-dried. Thereby, 10K purified particles (102.4 mg) were obtained. The filtrates washed with an Amicon (registered trademark) 10K filter were sequentially subjected to centrifugal separation at 10°C and 5,800 rpm for 60 minutes using the Amicon (registered trademark) 3K filter. Water was further added to the resultant product, and the mixture was subjected to centrifugal separation at 10°C and 5,800 rpm for 60 minutes. The concentrated liquid was filtered through a membrane (0.2 and freeze-dried. Thereby, 3K purified particles (41.2 mg) were obtained.

**[Table 1]**

| PEx | PSyn | Str | Data 1 |
|---|---|---|---|
| 1 | P1 | 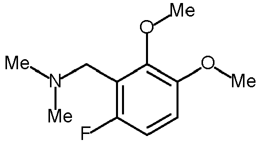 | ESI+:214 |
| 2 | P2 | 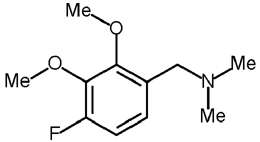 | ESI+:214 |
| 3 | P3 | 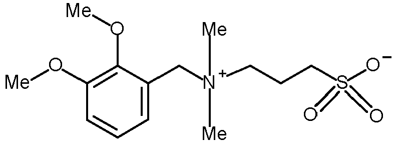 | ESI+:318 |
| 4 | P4 | 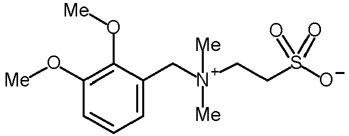 | ESI+:304 |
| 5 | P5 | 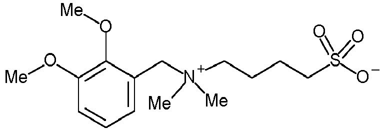 | ESI+:332 |
| 6 | P6 | 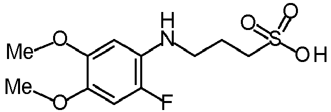 | ESI+:294 |
| 7 | P7 | 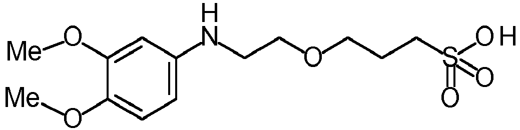 | ESI+:320 |
| 8 | P8 | 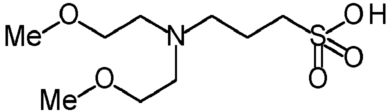 | ESI+:256 |

**[Table 2]**

| PEx | PSyn | Str | Data 1 |
|---|---|---|---|
| 9 | P9 | 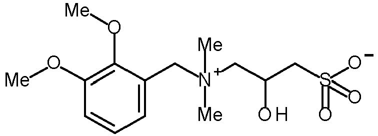 | ESI+:334 |
| 10 | P10 | 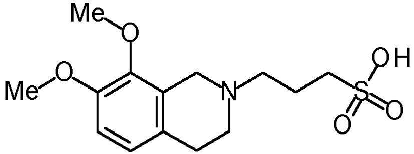 | ESI+:316 |
| 11 | P11 | 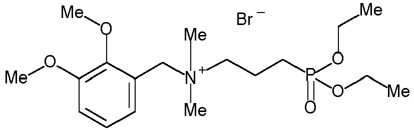 | ESI(M+)+:374 |
| 12 | P12 | 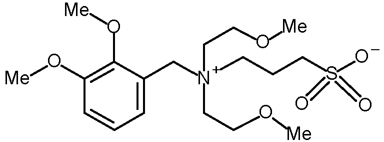 | ESI+:406 |
| 13 | P13 | 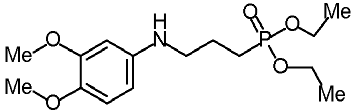 | ESI+:332 |
| 14 | P14 | 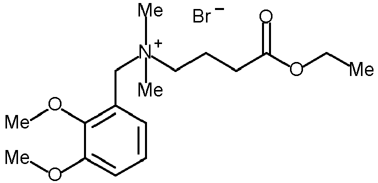 | ESI(M+)+:310 |
| 15 | P15 | 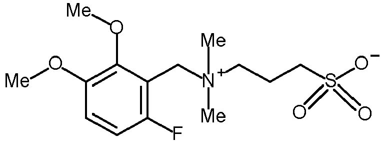 | ESI+:336 |

**[Table 3]**

| PEx | PSyn | Str | Data 1 |
|---|---|---|---|
| 16 | P16 | 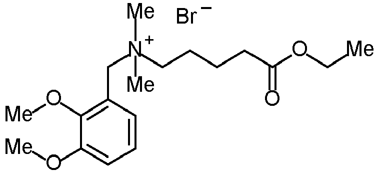 | APCI/ESI(M+)+:32 4 |
| 17 | P17 | 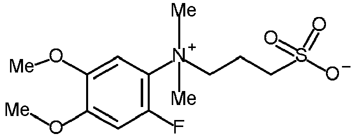 | ESI+:322 |
| 18 | P18 | 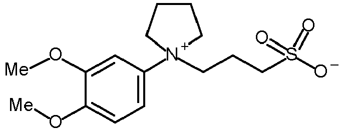 | ESI+:330 |
| 19 | P19 | 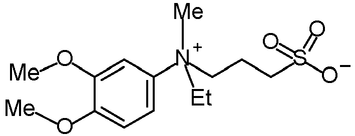 | ESI+:318 |
| 20 | P20 | 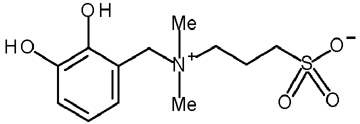 | ESI+:290 |
| | | | NMR-D: 2.04-2.15 (2H, m), 2.44-2.48 (2H, m), 2.92 (6H, s), 3.39-3.45 (2H, m), 4.40 (2H, s), 6.73 (1H, dd, J = 7.6, 7.6Hz), 6.84 (1H, dd, J = 7.6, 1.5Hz), 6.92 (1H, dd, J = 7.6, 1.5Hz), 9.22 (1H, br s), 9.77 (1H, br s) |

**[Table 4]**

| PEx | PSyn | Str | Data 1 |
|---|---|---|---|
| 21 | P21 | 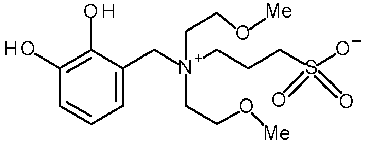 | ESI+:378 |
| 22 | P22 | 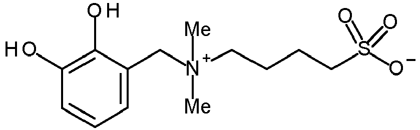 | ESI+:304 |
| | | | NMR-D: 1.56-1.65 (2H, m), 1.83-1.91 (2H, m), 2.45-2.50 (2H, m), 2.91 (6H, s), 3.24-3.31 (2H, m), 4.39 (2H, s), 6.74 (1H, dd, J = 8.0, 8.0Hz), 6.83 (1H, dd, J = 8.0, 1.5Hz), 6.93 (1H, dd, J = 8.0, 1.5Hz), 9.25 (1H, br s), 9.80 (1H, br s) |
| 23 | P23 | 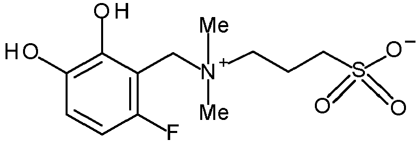 | ESI+:308 |
| | | | NMR-D: 2.03-2.13 (2H, m), 2.46 (2H, t, J = 7.1Hz), 2.96 (6H, s), 3.43-3.50 (2H, m), 4.43 (2H, s), 6.61-6.66 (1H, m), 6.92 (1H, dd, J = 8.8, 5.8Hz), 9.77 (1H, s), 9.83 (1H, s) |

**[Table 5]**

| PEx | PSyn | Str | Data 1 |
|---|---|---|---|
| 24 | P24 | 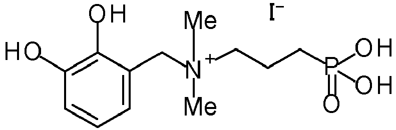 | ESI(M+)+:290 NMR-D: 1.51-1.60 (2H, m), 1.93-2.03 (2H, m), 2.94 (6H, s), 3.32-3.38 (2H, m), 4.42 (2H, s), 6.74 (1H, dd, J = 7.7, 7.7Hz), 6.84 (1H, dd, J = 7.7, 1.5Hz), 6.94 (1H, dd, J = 7.7, 1.5Hz), 9.21 (1H, br s), 9.83 (1H, br s) |
| 25 | P25 | 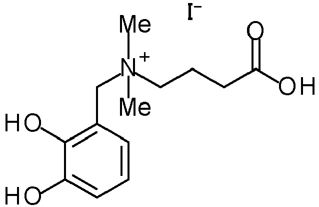 | ESI(M+)+:254 NMR-D: 1.97-2.04 (2H, m), 2.32 (2H, t, J = 7.2Hz), 2.95 (6H, s), 3.25-3.30 (2H, m), 4.42 (2H, s), 6.74 (1H, dd, J = 7.8, 7.8Hz), 6.85 (1H, dd, J = 1.3, 7.8Hz), 6.93 (1H, dd, J = 1.5, 7.8Hz), 9.25 (1H, s), 9.82 (1H, s), 12.33 (1H, s) |

**[Table 6]**

| PEx | PSyn | Str | Data 1 |
|---|---|---|---|
| 26 | P26 | 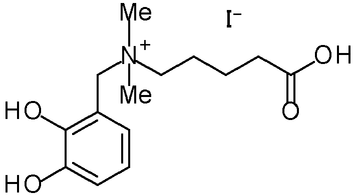 | ESI(M+)+:268 NMR-D: 1.51 (2H, quin, J = 7.5Hz), 1.75-1.85 (2H, m), 2.30 (2H, t, J = 7.3Hz), 2.93 (6H, s), 3.24-3.29 (2H, m), 4.40 (2H, s), 6.74 (1H, dd, J = 7.8, 7.8Hz), 6.83 (1H, dd, J = 1.5, 7.8Hz), 6.93 (1H, dd, J = 1.5, 7.8Hz), 9.25 (1H, s), 9.83 (1H, s), 12.13 (1H, s) |
| 27 | P27 | 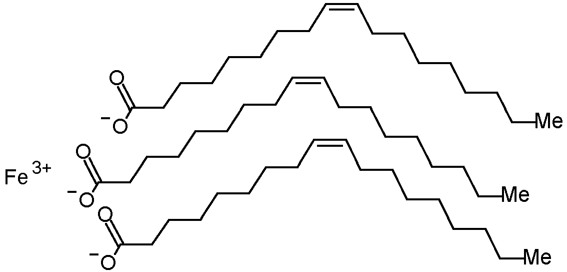 | ESI(M-)-:281 |
| 28 | P28 | 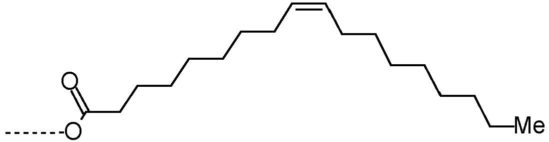 | |
| 29 | P1 | 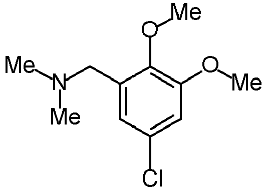 | ESI+:230 |

**[Table 7]**

| PEx | PSyn | Str | Data 1 |
|---|---|---|---|
| 30 | P3 | 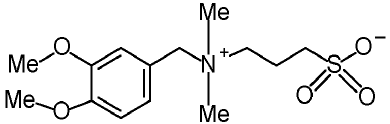 | ESI+:318 |
| 31 | P3 | 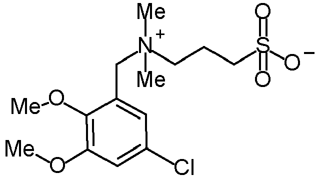 | ESI+:352 |
| 32 | P3 | 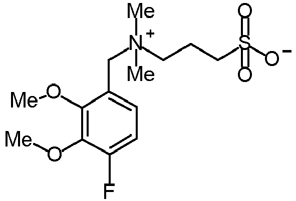 | ESI+:336 |
| 33 | P33 | 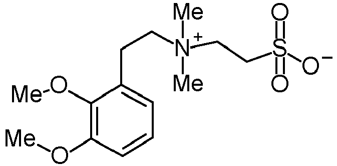 | ESI+:318 |
| 34 | P5 | 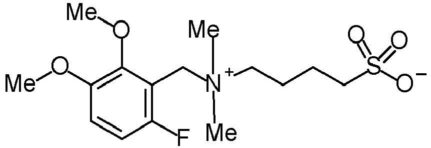 | ESI+:350 |
| 35 | P35 | 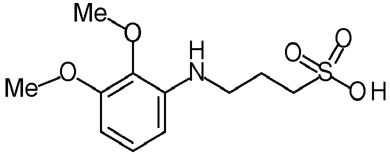 | ESI+:276 |
| 36 | P6 | 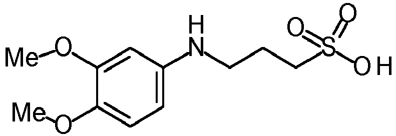 | ESI+:276 |

**[Table 8]**

| PEx | PSyn | Str | Data 1 |
|---|---|---|---|
| 37 | P6 | 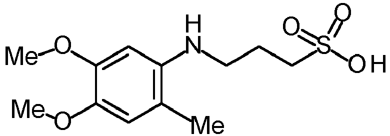 | ESI+:290 |
| 38 | P38 | 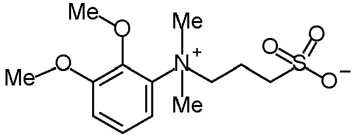 | ESI+:304 |
| 39 | P17 | 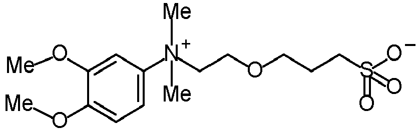 | ESI+:348 |
| 40 | P17 | 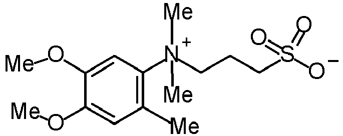 | ESI+:318 |
| 41 | P17 | 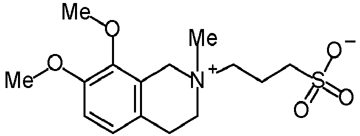 | ESI+:330 |
| 42 | P17 | 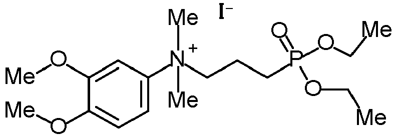 | ESI(M+)+:360 |
| 43 | P43 | 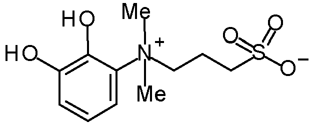 | ESI+:276 NMR-D: 1.57-1.63 (2H, m), 2.36 (2H, t, J = 7.5 Hz), 3.59 (6H, s), 4.06-4.11 (2H, m), 6.84 (1H, dd, J = 8.4, 8.4 Hz), 7.02-7.08 (2H, m), 10.21 (1H, s), 10.39 (1H, s) |

**[Table 9]**

| PEx | PSyn | Str | Data 1 |
|---|---|---|---|
| 44 | P20 | 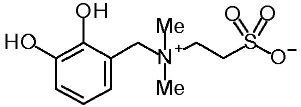 | ESI+:276 |
| | | | NMR-D: 2.94 (6H, s), 3.01-3.07 (2H, m), 3.53-3.58 (2H, m), 4.44 (2H, s), 6.73 (1H, dd, J = 7.7, 7.7Hz), 6.84 (1H, dd, J = 7.7, 1.5Hz), 6.92 (1H, dd, J = 7.7, 1.5Hz), 9.21 (1H, br s), 9.77 (1H, br s) |
| 45 | P20 | 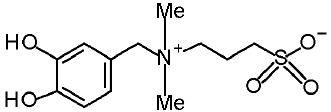 | ESI+:290 |
| 46 | P20 | 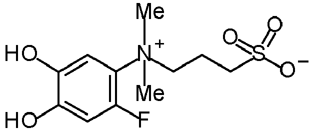 | ESI+:294 |
| 47 | P20 | 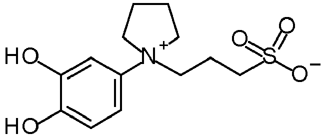 | ESI+:302 |
| 48 | P20 | 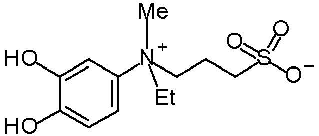 | ESI+:290 |

**[Table 10]**

| PEx | PSyn | Str | Data 1 |
|---|---|---|---|
| 49 | P20 | 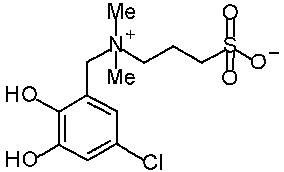 | ESI+:324 |
| 50 | P50 | 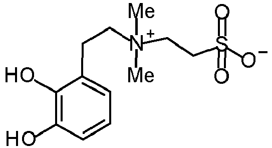 | ESI+:290 NMR-D: 2.90-2.96 (2H, m), 2.96-3.01 (2H, m), 3.10 (6H, s), 3.34-3.39 (2H, m), 3.59-3.65 (2H, m), 6.58-6.64 (2H, m), 6.70 (1H, dd, J = 2.2, 7.2 Hz), 8.57 (1H, br s), 9.33 (1H, br s) |

**[Table 11]**

| PEx | PSyn | Str | Data 1 |
|---|---|---|---|
| 51 | P20 | 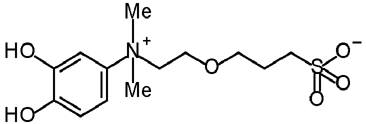 | ESI+:320 |
| 52 | P20 | 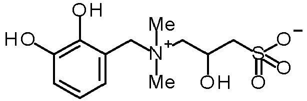 | ESI+:306 |
| 53 | P20 | 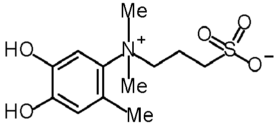 | ESI+:290 |
| 54 | P20 | 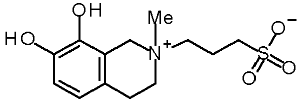 | ESI+:302 |
| 55 | P20 | 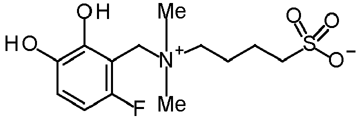 | ESI+:322 |
| | | | NMR-D: 1.63 (2H, quin, J = 7.5Hz), 1.82-1.90 (2H, m), 2.45-2.49 (2H, m), 2.94 (6H, s), 3.32-3.37 (2H, m), 4.43 (2H, s), 6.61-6.66 (1H, m), 6.93 (1H, dd, J = 8.8, 6.0Hz), 9.76-9.89 (2H, m) |
| 56 | P20 | 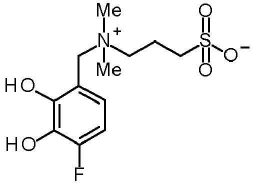 | ESI+:308 |
| 57 | P20 | 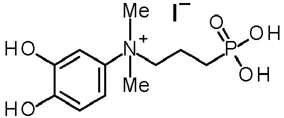 | ESI(M+)+:276 |

**[Table 12]**

| PEx | PSyn | Str | Data 1 |
|---|---|---|---|
| 58 | P58 | 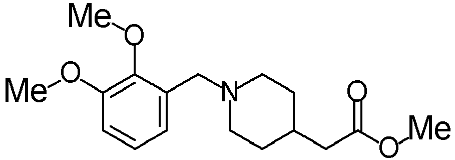 | ESI+:308 |
| 59 | P59 | 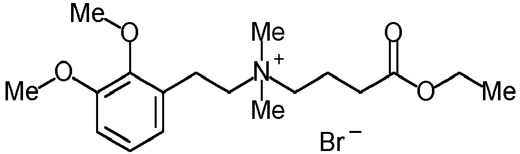 | ESI+:324.5(M+) |
| 60 | P60 | 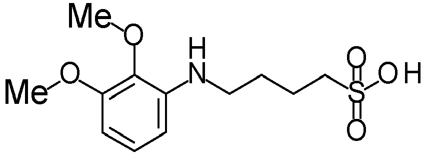 | ESI+:290 |
| 61 | P61 | 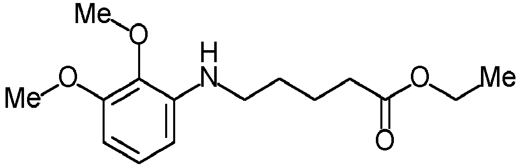 | ESI+:282 |
| 62 | P62 | 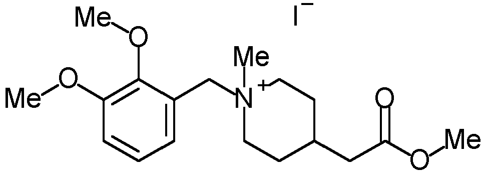 | ESI+:322 (M+) |
| 63 | P63 | 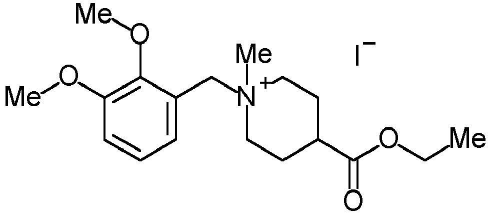 | ESI+:322 (M+) |

**[Table 13]**

| PEx | PSyn | Str | Data 1 |
|---|---|---|---|
| 64 | P64 | 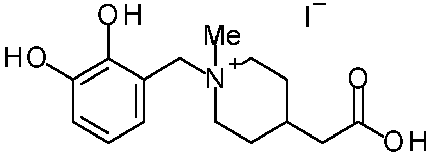 | ESI+:280 (M+) NMR-D: 1.60-1.72 (2H, m), 1.75-1.82 (2H, m), 1.83-1.97 (1H, m), 2.28, 2.36 (2H, d, J = 6.9 Hz), 2.83, 2.93 (3H, s), 3.26-3.48 (4H, m), 4.45, 4.50 (2H, s), 6.74 (1H, dd, J = 7.8, 7.8 Hz), 6.84 (1H, dd, J = 1.4, 7.8 Hz), 6.93 (1H, dd, J = 1.4, 7.8 Hz), 9.22 (1H, s), 9.81 (1H, s), 12.19 (1H, br) |
| 65 | P65 | 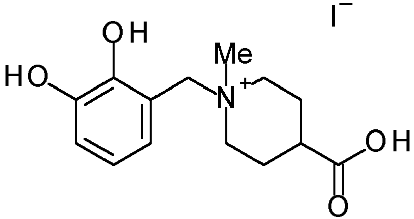 | ESI+:266 (M+) NMR-D: 1.90-2.05 (4H, m), 2.52-2.60 (1H, m), 2.93 (3H, s), 3.34-3.46 (4H, m), 4.46 (2H, s), 6.74 (1H, dd, J = 7.7, 7.7 Hz), 6.83 (1H, dd, J = 7.7, 1.5 Hz) 6.93 (1H, dd, J = 7.7, 1.5 Hz), 9.25 (1H, s), 9.83 (1H, s), 12.55 (1H, s) |

**[Table 14]**

| PEx | PSyn | Str | Data 1 |
|---|---|---|---|
| 66 | P66 | 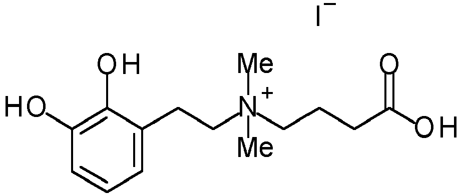 | ESI+:268 (M+) NMR-D: 1.89-1.97 (2H, m), 2.33 (2H, t, J = 7.0 Hz), 2.92-2.97 (2H, m), 3.09 (6H, s), 3.31-3.42 (4H, m), 6.60 (1H, dd, J = 7.7, 7.7 Hz), 6.64 (1H, dd, J = 1.8, 7.7 Hz), 6.71 (1H, dd, J = 1.8, 7.7 Hz), 8.59 (1H, s), 9.39 (1H, s), 12.36 (1H, br) |
| 67 | P17 | 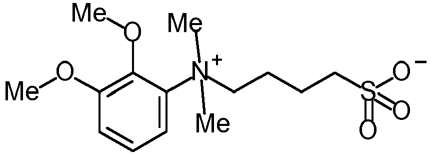 | ESI+:318 |
| 68 | P20 | 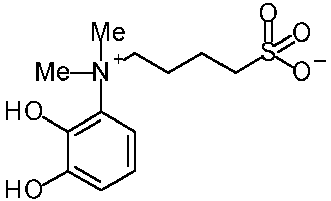 | ESI+:290 |
| 69 | P17 | 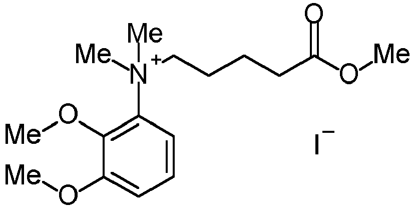 | ESI+:296 (M+) |

**[Table 15]**

| PEx | PSyn | Str | Data 1 |
|---|---|---|---|
| 70 | P25 | 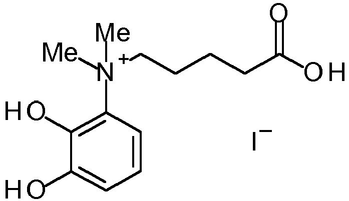 | ESI+:254 (M+) |
| 71 | P14 | 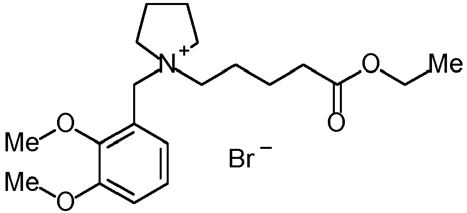 | ESI+:350 (M+) |
| 72 | P25 | 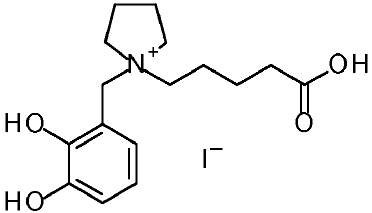 | ESI+:294 (M+) |
| 73 | P14 | 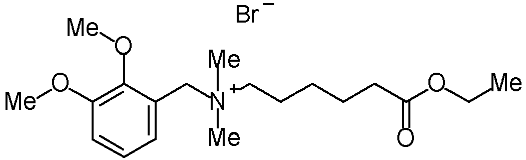 | ESI+:338 (M+) |
| 74 | P25 | 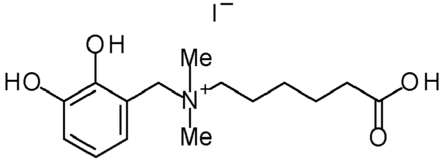 | ESI+:282 (M+) |

**[Table 16]**

| Ex Str Data 2 | | |
|---|---|---|
| 7-1 | 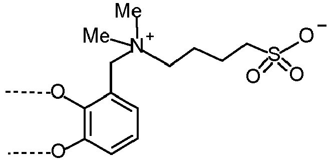 | SEC(min): 11.2-11.5 (3K) |
| | | SEC(min): 10.7-11.1 (10K) |
| 7-2 | 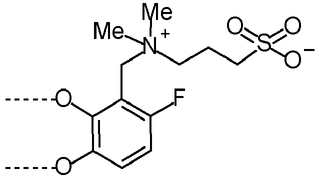 | SEC(min): 11.2-11.4 (3K) |
| | | SEC(min): 10.8-10.9 (10K) |
| 7-3 | 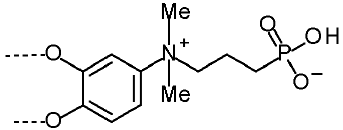 | |

### [Evaluation test of particle diameter of nanoparticles]

The particle diameters of the nanoparticles produced by the phase transfer catalyst method of the present invention were evaluated, and were further compared with the nanoparticles obtained by the known production method described in the comparative examples.

Relative sizes of the nanoparticles were measured using Size Exclusion Chromatography (SEC).

SEC is an analytical method in which a sample is poured into a column filled with a carrier having pores, and sizes of the sample are estimated by the time until it flows out. Large agglomerates flow out quickly because they do not enter the pores of the carrier, and small nanoparticles flow out slowly because they pass through the pores of the carrier and the route to flow out becomes long, and thereby relative sizes can be measured using standard particles.

Under the following SEC conditions, measurement was performed twice on the 3K purified nanoparticles and 10K purified nanoparticles produced by the MEAA method of Comparative Example 1, and the 3K purified nanoparticles and 10K purified nanoparticles of Example 7-1 which were produced by the phase transfer catalyst method using the same ligand as that of Comparative Example 1; and twice on the 3K purified nanoparticles and 10K purified nanoparticles produced by the MEAA method of Comparative Example 2, and the 3K purified nanoparticles and 10K purified nanoparticles of Example 7-2 which were produced by the phase transfer catalyst method using the same ligand as that of Comparative Example 2.

### <SEC conditions>

Flow velocity: 0.3 mL/min.
Eluent: PBS (pH 7.4)
Column: Shodex KW403-4F (4.6 x 300 mm)
Detector: UV 280nm

The results are shown below.

An outflow time of SEC of the 3K purified nanoparticles of Comparative Example 1 was between 10.8 and 11.4 minutes, and a ratio with respect to an outflow time (9.4 to 10.2 minutes) of ovalbumin, which is the standard, was 1.11 to 1.14. Furthermore, an outflow time of SEC of the 10K purified nanoparticles of Comparative Example 1 was between 10.6 and 11.0 minutes, and a ratio with respect to an outflow time (9.4 to 10.2 minutes) of ovalbumin, which is the standard, was 1.07 to 1.10.

An outflow time of SEC of the 3K purified nanoparticles of Example 7-1 was between 11.2 and 11.5 minutes, and a ratio with respect to an outflow time (9.4 to 10.2 minutes) of ovalbumin, which is the standard, was 1.12 to 1.14. Furthermore, an outflow time of SEC of the 10K purified nanoparticles of Example 7-1 was between 10.7 and 11.1 minutes, and a ratio with respect to an outflow time (9.4 to 10.2 minutes) of ovalbumin, which is the standard, was 1.06 to 1.09.

An outflow time of SEC of the 3K purified nanoparticles of Comparative Example 2 was between 11.3 and 11.4 minutes, and a ratio with respect to an outflow time (9.4 to 10.2 minutes) of ovalbumin, which is the standard, was 1.15 to 1.16. Furthermore, an outflow time of SEC of the 10K purified nanoparticles of Comparative Example 2 was between 10.5 and 10.8 minutes, and a ratio with respect to an outflow time (9.4 to 10.2 minutes) of ovalbumin, which is the standard, was 1.07 to 1.10.

An outflow time of SEC of the 3K purified nanoparticles of Example 7-2 was between 11.2 and 11.4 minutes, and a ratio with respect to an outflow time (9.4 to 10.2 minutes) of ovalbumin, which is the standard, was 1.12 to 1.14. Furthermore, an outflow time of SEC of the 10K purified nanoparticles of Example 7-2 was between 10.8 and 10.9 minutes, and a ratio with respect to an outflow time (9.4 to 10.2 minutes) of ovalbumin, which is the standard, was 1.08 to 1.10.

Based on the above descriptions, from the ratio of the outflow time with respect to the standard, it was confirmed that the nanoparticles obtained in the above examples had a smaller particle diameter than that of the standard (ovalbumin: particle diameter 6.1 nm). Furthermore, from the comparison of the outflow time of SEC with the comparative examples, it was confirmed that, even when the production methods are different, the nanoparticles having almost the same particle diameter can be obtained in a case of using the same zwitterionic ligand.

### Industrial Applicability

The present invention relates to a production method, which is suitable in industrial production, for a nanoparticle having a metal particle which contains iron oxide to which one or more hydrophilic ligands are coordination bonded, where the nanoparticle is expected to be used as an MRI contrast agent and the like.

## Claims

1. A method for producing a nanoparticle having a metal particle which contains iron oxide to which one or more hydrophilic ligands are coordination bonded, the method comprising
reacting an iron oxide nanoparticle having a surface to which a hydrophobic ligand is coordination bonded, with a hydrophilic ligand in a two-layer solvent of an organic layer and an aqueous layer in the presence of a phase transfer catalyst.

2. The production method according to claim 1,
wherein the hydrophilic ligand is a zwitterionic ligand containing a tertiary ammonium cation or a quaternary ammonium cation as a positively charged moiety, and containing a carboxylate anion, a sulfonate anion, a sulfinate anion, a phosphonate anion, a phosphate anion, or a phosphinate anion as a negatively charged moiety.

3. The production method according to claim 2,
wherein the hydrophilic ligand is a zwitterionic ligand having a catechol structure as a partial structure.

4. The production method according to claim 3,
wherein the hydrophobic ligand is a C₁₀₋₂₂ fatty acid, and the hydrophilic ligand is a zwitterionic ligand shown by the formula (I), 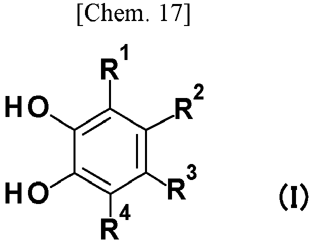
(in the formula, one of R¹ and R² is a group shown by the formula (a) or the formula (b), and the other is H, lower alkyl, O-lower alkyl, or halogen, 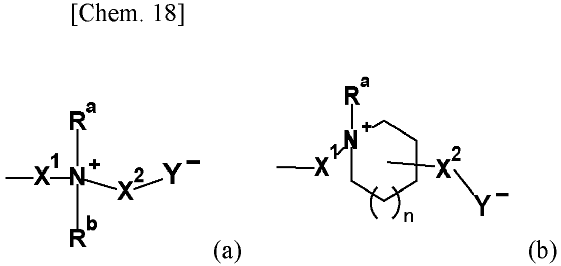
X¹ is a bond or methylene, and when R¹ is the group shown by the formula (a), X¹ may be ethylene,
X² is C₁₋₅ alkylene which may be substituted with OH, or C₁₋₂ alkylene-O-C₁₋₃ alkylene, and when R¹ is the group shown by the formula (b), X² may be a bond,
R^{a} and R^{b} are the same as or different from each other and each are C₁₋₃ alkyl or C₁₋₃ alkylene-O-C₁₋₂ alkyl, or R^{a} and R^{b} are integrated with a quaternary nitrogen atom to which R^{a} and R^{b} are bonded to form a 5- or 6-membered nitrogen-containing saturated heterocycle,
Y⁻ is SO₃⁻, HPO₃⁻, or CO₂⁻,
R³ and R⁴ are the same as or different from each other and each are H, C₁₋₃ alkyl, O-C₁₋₃ alkyl, or halogen,
n is an integer of 0 to 2, and
i) when R¹ is the group shown by the formula (a) and X¹ is methylene, R² and R^{a} or R^{b} may be integrated with each other to form ethylene,
ii) when R¹ is the group shown by the formula (a) and X¹ is ethylene, R² and R^{a} or R^{b} may be integrated with each other to form methylene, and
iii) when R² is the group shown by the formula (a) and X¹ is methylene, R³ and R^{a} or R^{b} may be integrated with each other to form ethylene)

5. The production method according to claim 4,
wherein the hydrophilic ligand is the zwitterionic ligand in which one of R¹ and R² is the group shown by the formula (a), and the other is H, lower alkyl, O-lower alkyl, or halogen.

6. The production method according to claim 5, which is a method for producing an iron oxide nanoparticle having a surface to which one or more hydrophilic ligands, which are zwitterionic ligands shown by the formula (Ia), are coordination bonded, the method comprising
reacting an iron oxide nanoparticle having a surface to which a hydrophobic ligand, which is a C₁₀₋₂₂ fatty acid, is coordination bonded, with a hydrophilic ligand, which is a zwitterionic ligand shown by the formula (Ia), in a two-layer solvent of an organic layer and an aqueous layer in the presence of a phase transfer catalyst to exchange a ligand to be bonded from a hydrophobic ligand to a hydrophilic ligand, 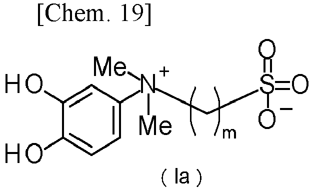 (in the formula, m is an integer of 1 to 4).

7. The production method according to claim 6,
wherein the hydrophilic ligand is the zwitterionic ligand in which m is 3 in the formula (Ia).

8. The production method according to any one of claims 1 to 7,
wherein the phase transfer catalyst is one or more catalysts selected from the group consisting of quaternary ammonium salts and quaternary phosphonium salts.

9. The production method according to claim 8,
wherein the hydrophobic ligand is oleic acid and/or stearic acid, and the phase transfer catalyst is one or more catalysts selected from the group consisting of tetrabutylammonium salts, trioctylmethylammonium salts, benzyldimethyloctadecylammonium salts, and benzyltributylammonium salts.

10. The production method according to claim 9,
wherein the hydrophobic ligand is oleic acid, and the phase transfer catalyst is one or more catalysts selected from the group consisting of tetrabutylammonium halides and benzyltributylammonium halides.

11. The production method according to claim 10,
wherein the phase transfer catalyst is one or more catalysts selected from the group consisting of tetrabutylammonium bromide, tetrabutylammonium chloride, tetrabutylammonium fluoride, and benzyltributylammonium bromide.

12. The production method according to any one of claims 1 to 11,
wherein the iron oxide nanoparticle having a surface to which a hydrophobic ligand is coordination bonded is reacted with 1 to 10 wt (weight ratio) of hydrophilic ligands with respect to the iron oxide nanoparticle having a surface to which a hydrophobic ligand is coordination bonded, in the presence of 0.1 to 10 wt (weight ratio) of the phase transfer catalyst with respect to the iron oxide nanoparticle having a surface to which a hydrophobic ligand is coordination bonded.

13. The production method according to any one of claims 1 to 12,
wherein the iron oxide nanoparticle having a surface to which a hydrophobic ligand is coordination bonded is an iron oxide nanoparticle having a surface to which a C₁₀₋₂₂ fatty acid is coordination bonded and produced by a method including:
i) preparing a first solution at 150°C to 190°C, and a second solution at 0°C to 120°C containing an iron precursor that is an iron complex of a C₁₀₋₂₂ fatty acid, where any one or both of the first solution and the second solution contain one or more surfactants selected from the group consisting of C₁₀₋₂₂ fatty alcohols, C₁₀₋₂₂ fatty acids, and C₁₀₋₂₂ fatty amines;
ii) maintaining the first solution at 150°C to 190°C, and dropwise adding the second solution into the first solution; and
iii) reacting the mixed solution at 150°C to 190°C.

14. The production method according to claim 13,
wherein the iron precursor is an iron oleate complex or an iron stearate complex.

15. The production method according to claim 14,
wherein the surfactant is one or more surfactants selected from the group consisting of oleyl alcohol, oleic acid, and oleylamine.

16. A method for producing an iron oxide nanoparticle having a surface to which a C₁₀₋₂₂ fatty acid is coordination bonded, the method comprising:
i) preparing a first solution at 150°C to 190°C, and a second solution at 0°C to 120°C containing an iron precursor that is an iron complex of a C₁₀₋₂₂ fatty acid, where any one or both of the first solution and the second solution contain one or more surfactants selected from the group consisting of C₁₀₋₂₂ fatty alcohols, C₁₀₋₂₂ fatty acids, and C₁₀₋₂₂ fatty amines;
ii) maintaining the first solution at 150°C to 190°C, and dropwise adding the second solution into the first solution; and
iii) reacting the mixed solution at 150°C to 190°C.

17. The production method according to claim 16,
wherein the iron precursor is an iron oleate complex or an iron stearate complex, and the surfactant is one or more surfactants selected from the group consisting of oleyl alcohol, oleic acid, and oleylamine.

18. The production method according to claim 16 comprising:
i) preparing a first solution at 160°C to 180°C, and a second solution at 0°C to 100°C containing an iron oleate complex, where the first solution contains one or more surfactants selected from the group consisting of oleyl alcohol and oleylamine, and the second solution contains oleic acid or does not contain the surfactant;
ii) maintaining the first solution at 160°C to 180°C, and dropwise adding the second solution into the first solution for 0.1 to 3 hours; and
iii) reacting the mixed solution at 160°C to 180°C for 0.5 to 5 hours.

19. The production method according to claim 18,
wherein in i), the first solution contains 0.5 to 5 wt (weight ratio) of oleyl alcohol with respect to the iron oleate complex, and the second solution contains 0.1 to 2 wt (weight ratio) of oleic acid with respect to the iron oleate complex or does not contain the surfactant.

20. The production method according to any one of claims 16 to 19,
wherein in i), the first solution further contains a desiccant.
